# EUROPÄISCHE PATENTSCHRIFT

(11) **EP 0 806 932 B1**
(45) Veröffentlichungstag und Bekanntmachung des Hinweises auf die Patenterteilung: **06.05.1999**
(21) Anmeldenummer: 96903978.3
(22) Anmeldetag: 05.02.1996
(51) Int. Cl.: A61K 6/06, A61C 5/08, A61C 5/10, A61K 6/08

(54) **ZAHNRESTAURATIONS- ODER PROTHESENTEIL AUS KERAMIKMATERIAL SOWIE VERFAHREN ZU SEINER HERSTELLUNG**
TOOTH RESTORATION AND PROSTHESIS COMPONENT MADE FROM CERAMIC MATERIAL AND A METHOD OF MANUFACTURING THE SAME.
ELEMENT DE RESTAURATION DENTAIRE ET DE PROTHESE EN MATERIAU CERAMIQUE ET SON PROCEDE DE PRODUCTION

(30) Priorität: 05.02.1995 DE 19503637
(43) Veröffentlichungstag der Anmeldung: 19.11.1997
(73) Patentinhaber: HAHN, Rainer, D-72074 Tubingen (DE)
(72) Erfinder: HAHN, Rainer, D-72074 Tubingen (DE)
(74) Vertreter: Ostertag, Reinhard
(86) Internationale Anmeldenummer: EP9600460
(87) Internationale Veröffentlichungsnummer: WO9623481

(56) Entgegenhaltungen:
- EP-A- 0 580 565
- US-A- 4 813 874

## Beschreibung

Die Erfindung betrifft ein Zahnrestaurations- oder Prothesenteil aus Keramikmaterial gemäß dem Oberbegriff des Anspruches 1 bzw. 37.

In der nachstehenden Beschreibung wird der besseren Lesbarkeit halber überwiegend von Zahnrestaurationsteilen gesprochen; die Ausführungen gelten aber sinngemäß auch für andere Prothesenteile aus Keramikmaterial.

Die keramischen Zahnersatzwerkstoffe, aus denen derartige Zahnrestaurationsteile hergestellt werden, zeichnen sich im Prinzip durch hohe Druckfestigkeit, Verschleißbeständigkeit und gute Biokompatibilität aus. Sie neigen nur wenig zur Plaqueakkumulation, und aus ihnen können ästhetisch ansprechende Zahnrestaurationen mit individuellen Farb- und Transparenzverläufen hergestellt werden.

Bei derartigen Zahnrestaurationsteilen wird in der Praxis trotz der an sich gegebenen guten mechanischen Eigenschaften des Keramikmateriales häufig eine Rißbildung beobachtet, die zum Zerbrechen des Restaurationsteiles führen kann.

Durch die vorliegende Erfindung soll daher ein Zahnrestaurationsteil aus Keramikmaterial gemäß dem Oberbegriff des Anspruches 1 dahingehend weitergebildet werden, daß die Gefahr einer Rißbildung und eines rißbedingten Zerbrechens vermindert ist.

Diese Aufgabe ist erfindungsgemäß gelöst durch ein Zahnrestaurationsteil mit den im Anspruch 1 angegebenen Merkmalen bzw. durch Verfahren zum Herstellen eines solchen Zahnrestaurationsteiles mit den im Anspruch 19, 25, 27, 28 bzw. 29 angegebenen Merkmalen.

Ein erfindungsgemäßes Zahnrestaurationsteil hat den Vorteil, daß der Schutz gegen Rißbildung unabhängig davon erfolgt, wie oder mit welchem Befestigungsmaterial im einzelnen die Eingliederung durchgeführt wird, da die rißbildungverhindernde Schutzschicht vor Eingliederung unter genau vorgebbaren Bedingungen erzeugt wird. Das Zahnrestaurationsteil ist daher auch im Langzeiteinsatz zuverlässig.

Die Herstellung eines erfindungsgemäßen Zahnrestaurationsteiles kann unter Verwendung herkömmlicher Dentallaboranlagentechnik erfolgen.

Erfindungsgemäße Zahnrestaurationsteile zeichnen sich durch hohe Paßgenauigkeit und ansprechendes Erscheinungsbild aus.

Erfindungsgemäße Zahnrestaurationsteile lassen sich auch mit sehr geringer Wandstärke herstellen, wobei trotzdem eine zuverlässige Funktion und einwandfreie Farbgestaltung gewährleistet ist, so daß man bei der der Eingliederung vorausgehenden Präparation eines Zahnstumpfes den Abtrag an Zahnhartsubstanz sehr klein halten kann.

Durch die erfindungsgemäß auf dem Zahnrestaurationsteil vorgesehene Schutzschicht werden von den kritischen Stellen des Zahnrestaurationsteiles ausgehende Ermüdungsfrakturen vermieden. Zu solchen kann es bei ständiger Belastung unter antagonistischem Zahnkontakt bzw. durch Kaubeanspruchung kommen, da Kerbspannungseffekte eine Induktion von Mikrorissen herbeiführen und ein unterkritisches Wachstum derselben fördern, die auch durch hydrolytische Effekte, Feuchtigkeitsdehnung und Ionenaustauschmechanismen (Speichel, Dentinliquor) begünstigt werden. Obwohl die Elastizitätsgrenze des Keramikmateriales aufgrund rezeptorgesteuerter Schutzreflexe in der Regel unter Kaubelastung nicht überschritten wird, wird durch die andauernde subkritische Zug- oder Scherbeanspruchung eine Vergrößerung der vorhandenen Mikorrisse hervorgerufen. Nach Erreichen einer kritischen Mikrorißtiefe genügt dann eine vergleichsweise geringe Belastung des Zahnrestaurationsteiles um einen makroskopischen Riß und damit ein Versagen des Zahnrestaurationsteiles hervorzurufen.

Der Erzeugung und/oder dem Wachstum von Mikrorissen ausgehend von den kritischen Stellen des Zahnrestaurationsteiles wird durch die erfindungsgemäß vorgesehene Schutzschicht entgegengewirkt. Vorteilhafterweise weist die Schutzschicht einen dauerhaften Widerstand gegenüber den unter den Kaubeanspruchungen hervorgerufenen Zug- und/oder Scher- und/oder Druckbelastungen auf.

Die erfindungsgemäß auf kritischen Stellen des Zahnrestaurationsteiles vorgesehene Schutzschicht ist auch hydrolysestabil.

Gegen Rißbildung geschützte erfindungsgemäße Zahnrestaurationsteile können vollkeramische Zahnkronen, insbesondere Porzellanvollkronen als Einzelkrone oder im Kronenverbund, Brückenanker, Kronengerüste, Teilkronen oder Doppelkronen sein. Sie können aber auch Inlays sein, wobei die Schutzschicht längs der zentralen Längsfissur vorgesehen ist und vorzugsweise wenigstens teilweise in das der Oberfläche benachbarte Volumen des Inlays hineinragt. Vorteilhafte Weiterbildungen der Erfindung sind in abhängigen Ansprüchen wiedergegeben.

Gemäß einer bevorzugten Weiterbildung der Erfindung ist das Schutzschichtmaterial plastisch oder elastisch verformbar, wobei es vorzugsweise nach der Formgebung härtend oder erhärtbar ist. Die Schutzschicht ist spaltfrei auf die zu schützende Oberfläche des Zahnrestaurationsteiles aufgebracht und haftet wenigstens partiell kraftschlüssig an dieser, wodurch beanspruchsinduzierte Spannungskonzentrationen vermieden werden.

Gemäß einer weiteren Fortbildung der Erfindung kann die Schutzschicht zumindest teilweise aus keramischem Material bestehen. Hierzu können siliziumorganische Polymere auf die Innenfläche des Zahnrestaurationsteiles aufgetragen werden und nach dem Härten zumindest teilweise keramisiert werden. Dies kann z. B. durch Pyrolyse der Polymerphase in einer sauerstoffarmen Gasatmosphäre bei Temperaturen erfolgen, welche unterhalb der Plastifiziertemperatur des zur Herstellung des Zahnrestaurationsteiles verwendeten Keramikmateriales liegen.

Gemäß einer weiteren Fortbildung der Erfindung kann die Schutzschicht mindestens teilweise aus Kunststoff oder künstlichen oder natürlichen Harzen bestehen, die eine Auswahl aus generell geeigneten nichttoxischen, plastisch verformbaren und härtbaren oder erhärtenden Materialen darstellen. Hiervon sind besonders geeignet thermo- oder duroplastische Kunststoffe, Harze oder Polymere.

Besonders geeignete Schutzschichtmaterialien sind ferner Polysulfone, Acrylate oder siliziumorganische Polymere wie z. B. Polysiloxane.

Geeignete Schutzschichtmaterialien sind ferner Mischungen von zwei oder mehr unterschiedlichen Kunststoffen oder Harzen.

Die Schutzschicht kann ihrerseits aus verschiedenen Teilschichten aufgebaut sein.

Gemäß weiterer Fortbildung der Erfindung werden zumindest Teile des später die Schutzschicht bildenden Kunststoff- oder Harzvolumens mit inertem oder reaktivem Füllstoff versehen, wie sie beispielsweise in den dentalen Füllungskunststoffen oder fotopolymerisierbaren Glasionomerzementen oder neueren "Kompomeren" üblich sind. Durch Zugabe dieser zumeist anorganischen, nichtmetallischen Füllstoffe, deren Oberflächen vorzugsweise silanisiert sind, können die mechanischen Eigenschaften des resultierenden Komposits nach dessen Aushärtung verbessert werden. Über den Füllstoffgehalt läßt sich die Restschwindung bei der Aushärtung des Komposits beeinflussen. Auf diese Weise kann unter anderem die dadurch an der angrenzenden keramischen Grenzfläche induzierte Druckspannung kontrolliert werden. Diese Druckspannung wirkt sich zusätzlich günstig auf das Widerstandsverhalten der Schutzschicht gegenüber beanspruchungsinduzierten Rißöffungseffekten aus.

Bezüglich der Art, der Form, Konzentration oder Größenverteilung der verwendeten Füllstoffe wird angestrebt, daß bei hoher mechanischer Belastbarkeit, insbesondere hoher Zugelastizitätsgrenze, und möglichst geringer Sprödigkeit der Schutzschicht eine zum Aufbau von Druckspannungen in der angrenzenden Keramikoberfläche ausreichende Volumenschwindung bei der Aushärtung des Schutzschichtmateriales erhalten wird. Gleichzeitig soll die Schutzschicht eine ausreichende Steifigkeit und Druckfestigkeit aufweisen, um unter Kaubeanspruchung des verstärkten Zahnrestaurationsteiles nicht zerstört zu werden.

Vorzugsweise werden Schutzschichtmaterialien verwendet, die auch anorganische und/oder organische Partikel, Kurz- und/oder Langfasern und/oder daraus gefertigte Halbzeuge, insbesondere Fasergeflechte, enthalten. Hierdurch wird eine Verstärkung der Schutzschicht erhalten.

Als Füllstoffe können auch metallische Partikel und/oder metallische Kurz- oder Langfasern verwendet werden, z. B. aus Titan, wobei jedoch bezüglich der Farbe des Zahnrestaurationsteiles Zugeständnisse zu machen sind.

Es hat sich ferner bewährt, dem Schutzschichtmaterial zusätzlich foto- oder thermoaktivierbare Reaktionsinitiatoren und/oder Katalysatoren beizumischen, insbesondere bei Kunststoffen auf Acrylatbasis oder auf Basis siliziumorganischer Polymere.

Zur gezielten Polymerisationsaktivierung können dem zur Herstellung der Schutzschicht dienenden Material auch Polymerisationsinhibitoren und deren Antagonisten im Sinne von bei Raumtemperatur aushärtbaren Paste-Paste-Systemen zugegeben werden, welche unmittelbar vor Anwendung durchmischt werden.

Zur einfachen Verarbeitung der ungefüllten oder gefüllten oder faserverstärkten Materialien, aus denen die Schutzschicht hergestellt wird, hat sich deren Verwendung in Form von Pasten mit in der Zahntechnik oder Zahnheilkunde üblicher Konsistenz bzw. Viskosität bewährt. Alternativ zu Pasten können auch aus den Schutzschichtmaterialien hergestellte plastisch verformbare, z. B. folienartige Zwischenprodukte verwendet werden. Diese können z. B. die Form eines tablettenähnlichen Konfektionshalbzeuges haben.

Eine weitere vorteilhafte Art der Erzeugung der Schutzschicht ist diejenige ausgehend von teilweise vorpolymerisierten Halbzeugen (prepregs), die unter Wärmezufuhr zunächst ihre Viskosität deutlich verringern, wodurch sie einfach verformbar werden, und nach erfolgter Formgebung sich dann schnell verfestigen oder unter Einwirkung von Licht und/oder Wärme aushärten.

Die durch die erfindungsgemäß auf dem Zahnrestaurationsteil vorgesehene Schutzschicht garantierte Schutzwirkung ist unter anderem eine Folge der unterschiedlichen Elastizitätsmodule des eigentlichen Zahnrestaurationsteiles und der Schutzschicht. Die Schutzschicht bewirkt eine Druckspannung auf die beschichtete Keramikoberfläche, die unabhängig davon ist, wie das Zahnrestaurationsteil auf dem Zahnstumpf befestigt wird. Die bei Belastung von erfindungsgemäßen Zahnrestaurationsteilen, z.B. künstlichen Zahnkronen, erzeugten Spannungsspitzen sind an den kritischen Stellen des Zahnrestaurationsteiles lokalisiert und werden dort vollständig oder teilweise inaktiviert, z. B. durch elastische und/ oder plastische Verformung der Schutzschicht oder durch Versetzungen in der Polymerstruktur.

Verwendet man als Schutzschichtmaterial ungefüllte und/oder gefüllte, insbesondere partikel- oder faserverstärkte Kunststoffe, so hat das Schutzschichtmaterial verglichen mit dem Keramikmaterial einen wesentlich höheren Widerstand gegen belastungsinduzierte Rißöffungseffekte. Sollte sich dennoch ein Mikroriß bilden ist durch die Schutzschicht ein Widerstand gegen belastungsinduziertes Rißwachstum gegeben. Insbesondere der Widerstand gegen unterkritisches Rißwachstum ist verglichen zur reinen Dentalkeramik wesentlich größer.

Die zu schützenden kritischen Stellen des Zahnrestaurationsteiles sind durch die Schutzschicht ferner vor degradativen Hydrolyse- und/oder Korrosionseffekten geschützt, welche sonst durch an die Oberfläche gelangenden Dentinliquor bzw. im Sinne eines Leakage herandiffundierte Mundflüssigkeit hervorgerufen werden können.

Die Schutzwirkung der bei dem erfindungsgemäßen Zahnrestaurationsteil vorgesehenen Schutzschicht wird durch aus der Schwindung der Kunststoffphase im Zuge ihrer Polymerisation (Duroplast) oder Abkühlung (Thermoplast) resultierende Druckspannungen in der benachbarten keramischen Grenzfläche begünstigt. Der Aufbau solcher Druckspannungen an der inneren Grenzfläche zwischen Keramik und Schutzschicht sowie innerhalb der jeweils benachbarten Material-Randzonen beruht auf der wenigsten teilweise kraftschlüssigen Adaptation bzw. Adsorbtion der Schutzschicht auf dem Keramikmaterial. Die erzeugte Druckspannung wirkt auch unter Kaubeanspruchung induzierten Zugspannungen in den der Schutzschicht benachbarten Keramikbereichen entgegen. Die Schutzschicht stellt eine Versiegelung von möglicherweise fertigungs- oder bearbeitungsinduzierten Oberflächenfehlern der kritischen Stellen des Zahnrestaurationsteiles dar und vermindert so Kerbspannungseffekte. Dies gilt gleichermaßen für Zahnrestaurationsteile, die Zahnstümpfe übergreifen (Kronen und dergleichen) wie auch für Zahnrestaurationsteile, die in intrakoronale Zahnkavitäten eingesetzt sind (Inlays).

Nachstehend wird die Erfindung anhand von Ausführungsbeispielen unter Bezugnahme auf die Zeichnung näher erläutert. In dieser zeigen:
- Figuren 1 bis 7:: verschiedene Schritte bei der Herstellung eines keramischen Zahnrestaurationsteiles, nämlich:
- Figur 1:: die Herstellung eines Negativabdrucks von einem präparierten Zahnstumpf;
- Figur 2:: einen Schnitt durch ein vom Negativabdruck des Zahnstumpfes hergestelltes Positivmodell mit aufgesetzter Platzhalterschicht;
- Figur 3:: die Herstellung eines Negativabdruckes von dem die Platzhalterschicht tragenden Positivmodell;
- Figur 4:: die Modellierung einer keramischen Zahnkrone über einem Arbeitsmodell, welches mit dem in Figur 3 gezeigten Negativabdruck hergestellt ist;
- Figur 5:: das Herstellen einer Schutzschicht auf der Innenfläche der geformten und gebrannten Zahnkrone über dem Positivmodell des Zahnstumpfes;
- Figur 6:: eine teilweise geschnittene seitliche Ansicht der fertigen keramischen Zahnkrone;
- Figur 7:: einen Schnitt durch die auf den Zahnstumpf aufzementierte Zahnkrone;
- Figur 8:: einen vergrößerten Teilschnitt durch den Bereich VIII der eingegliederten Zahnkrone nach Figur 7;
- Figuren 9 bis 11:: ähnliche Schnittansichten wie Figur 8, in welchen abgewandelte keramische Zahnkronen gezeigt sind;
- Figur 12:: eine Ausschnittsvergrößerung des Bereiches XII der Zahnkrone nach Figur 11;
- Figuren 13 bis 17:: ähnliche Schnittansichten wie Figur 12, in welchen jedoch abgewandelte keramische Zahnkronen wiedergegeben sind;
- Figuren 18 und 19:: zwei Teilschritte bei einem abgewandelten Verfahren zur Herstellung einer eine Schutzschicht tragenden keramischen Zahnkrone; nämlich:

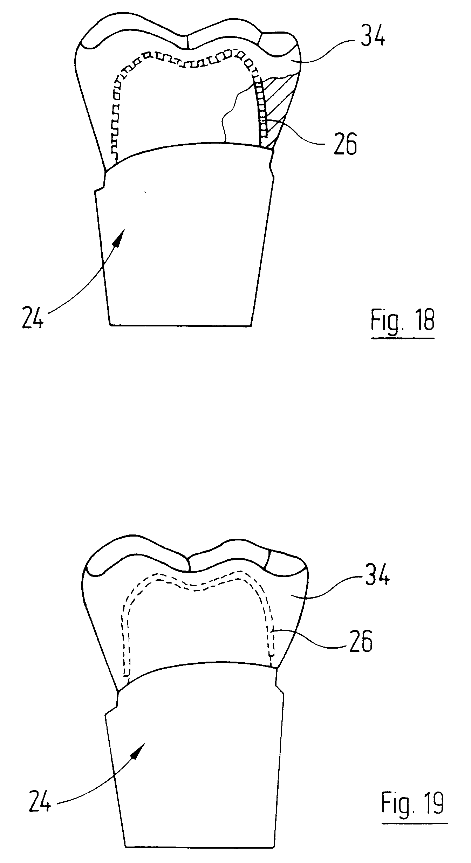

- Figur 18: : das Formen eines Modells des späteren keramischen Zahnrestaurationsteiles über einem Positivmodell des Zahnstumpfes, welches eine lösbare Platzhalterschicht trägt;
- Figur 19:: das Herstellen einer Schutzschicht auf dem gemäß Figur 18 geformten und anschließend hergestellten Zahnkronenrohling;

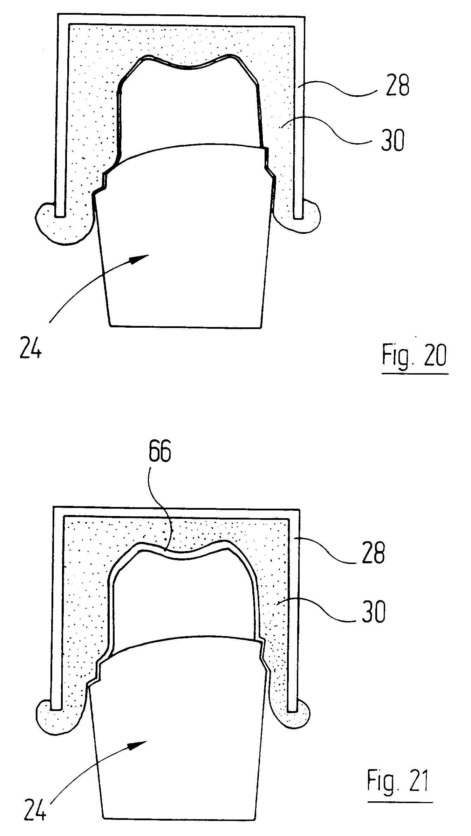

- Figuren 20 bis 22:: eine Alternative zur Herstellung eines Arbeitsmodelles, das bei der Formung des Keramikmateriales verwendet wird und gegenüber dem Zahnstumpf Übermaß aufweist; nämlich:
- Figur 20:: den Beginn der Herstellung eines Negativeabdruckes des vom Zahnstumpf gefertigten Positivmodelles;
- Figur 21:: die Verhältnisse nach Aushärten der für die Herstellung des Negativabdruckes verwendeten Abformmasse;

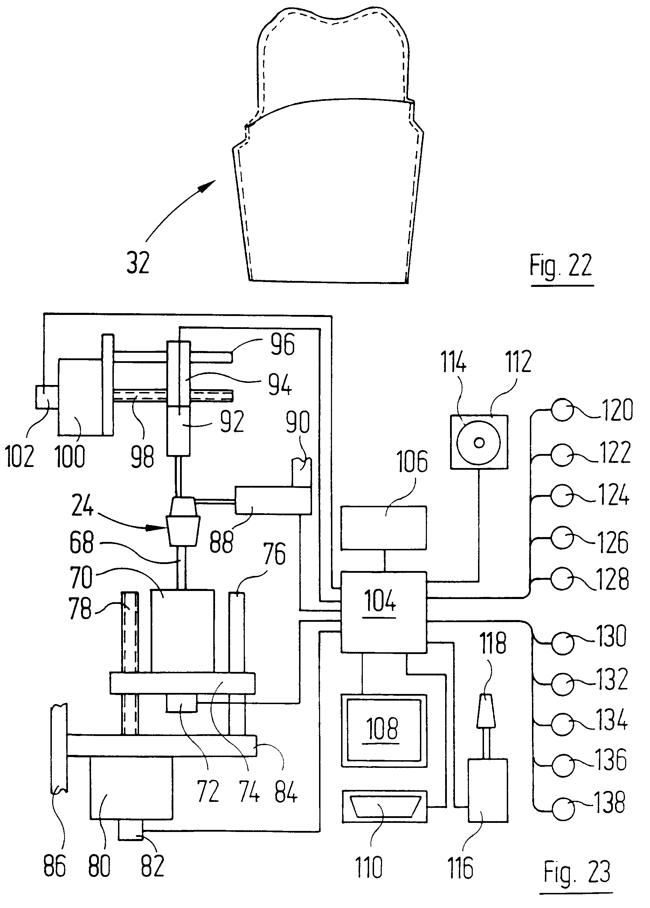

- Figur 22:: eine seitliche Ansicht des fertigen Arbeitsmodelles;
- Figur 23:: ein Blockschaltbild einer Vorrichtung zum Herstellen von Schutzschichtteilen mit der Außenkontur eines Positivmodelles des Zahnstumpfes entsprechender Innenkontur;

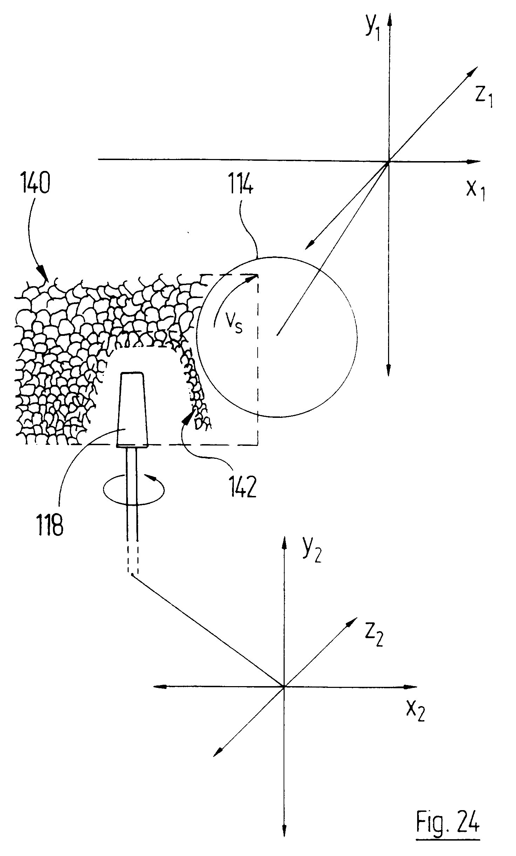

- Figur 24:: eine schematische Darstellung der Herstellung eines solchen Schutzschichtteiles, das Teil einer keramischen Zahnkrone werden soll;

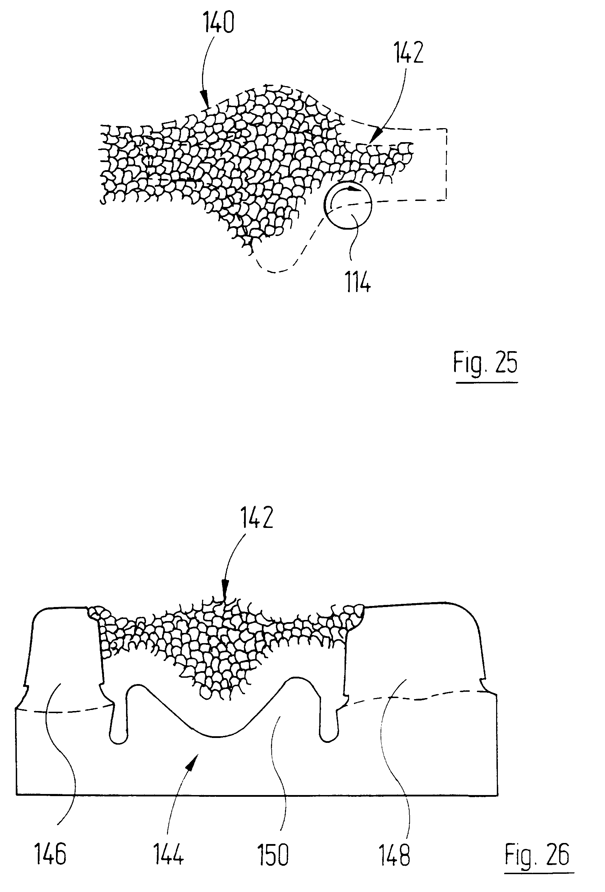

- Figur 25:: eine schematische Darstellung der Herstellung eines Schutzschichtteiles, welches Teil einer keramischen Brücke werden soll;
- Figur 26:: das Aufsetzen des Schutzschichteiles, welches gemäß Figur 25 hergestellt ist, auf ein Arbeitsmodell der die Brücke ankernden Zahnstümpfe;

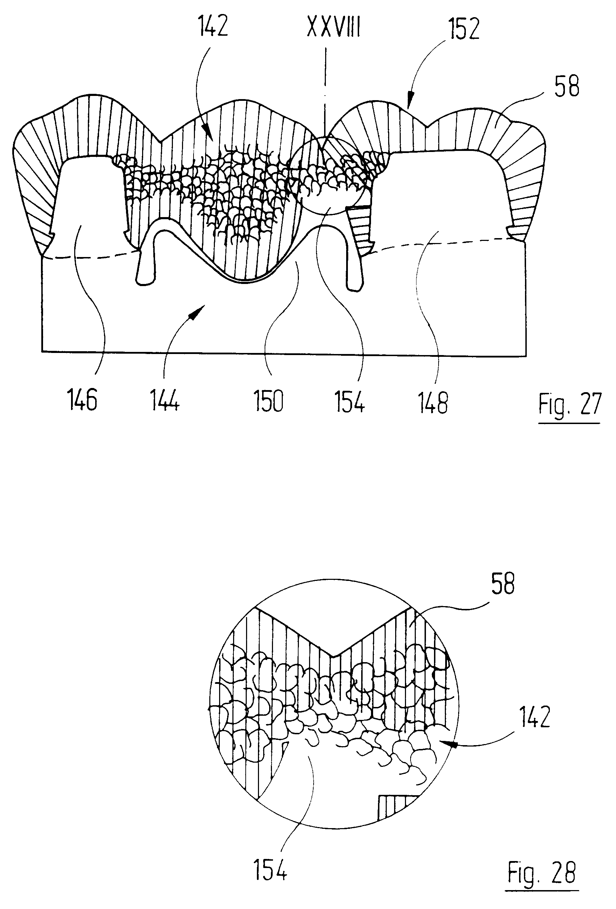

- Figur 27:: das Aufbauen von Keramikmaterial über dem Zahnstumpf-Arbeitsmodell und dem Schutzschichtteil;
- Figur 28:: einen vergrößerten Teilschnitt durch den Abschnitt XXVIII des Brückenrohlinges von Figur 27;

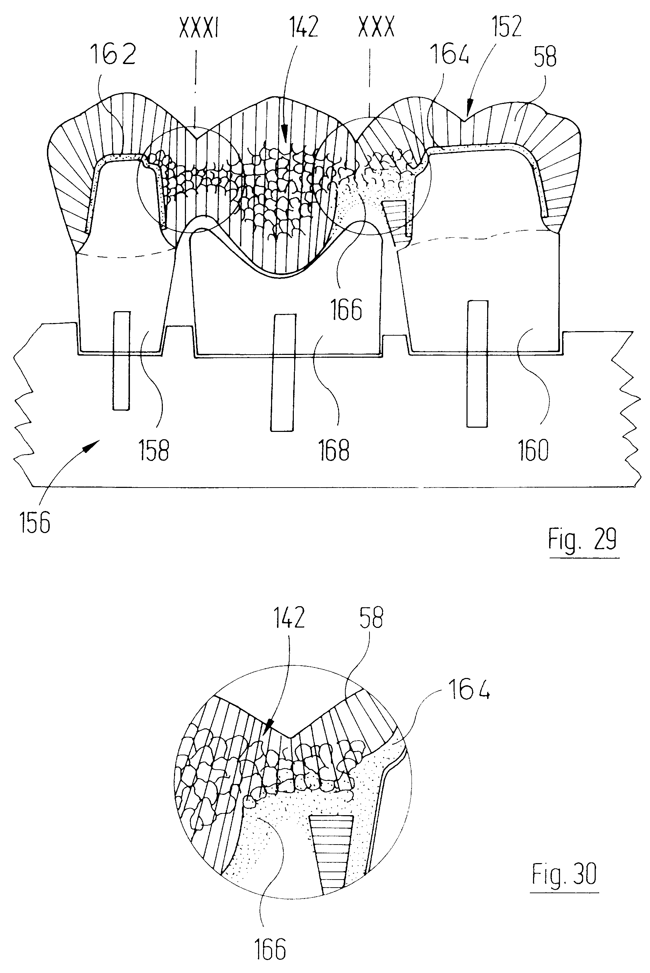

- Figur 29:: das Erzeugen von zusätzlichen Schutzschichten, die den präparierten Flächen der die Brücke ankernden Zahnstümpfe und weiteren besonders belasteten Stellen der Brücke zugeordnet sind;
- Figur 30:: eine erste Ausschnittsvergrößerung aus Figur 29, welche den dortigen Bereich XXX betrifft;

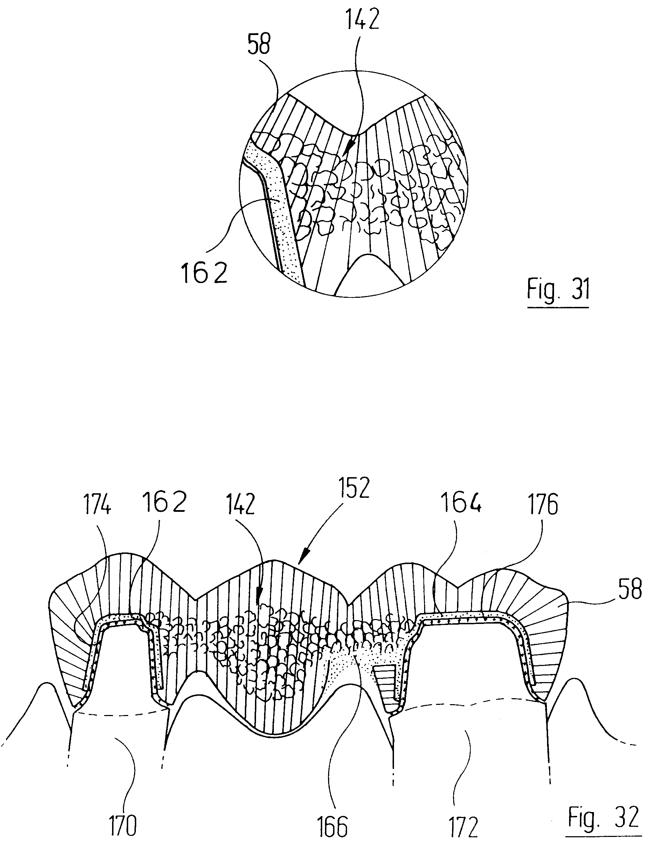

- Figur 31:: eine zweite Ausschnittsvergrößerung aus Figur 29, welche den dortigen Bereich XXXI betrifft,
- Figur 32:: einen Schnitt durch die eingegliederte keramische Brücke;

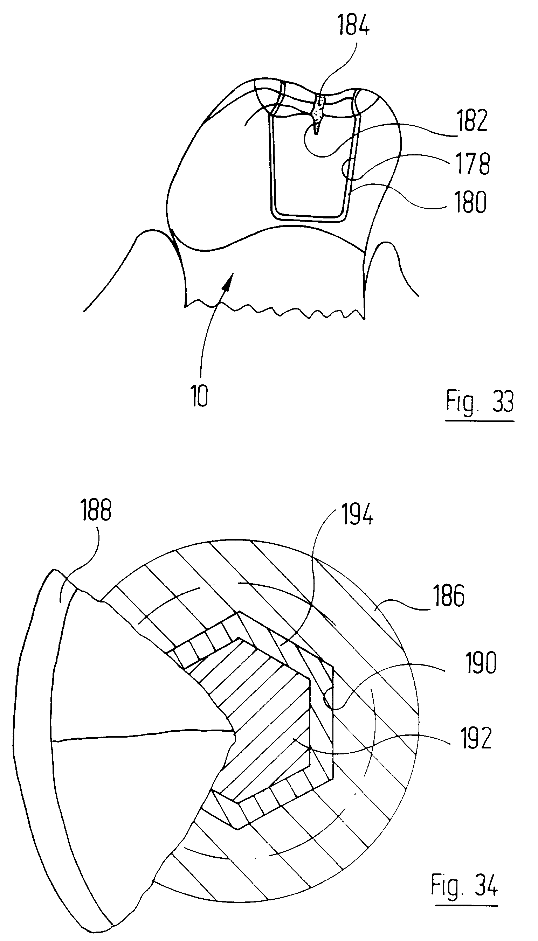

- Figur 33:: eine seitliche Ansicht eines eingegliederten Inlays, welches mit einer eine Rißbildung verhindernden Schutzschicht versehen ist; und
- Figur 34:: einen transversalen Schnitt durch die Verbindungsstelle eines Brückenanker-Implantates und eines mit diesem verbundenen Zahnrestaurationsteiles.

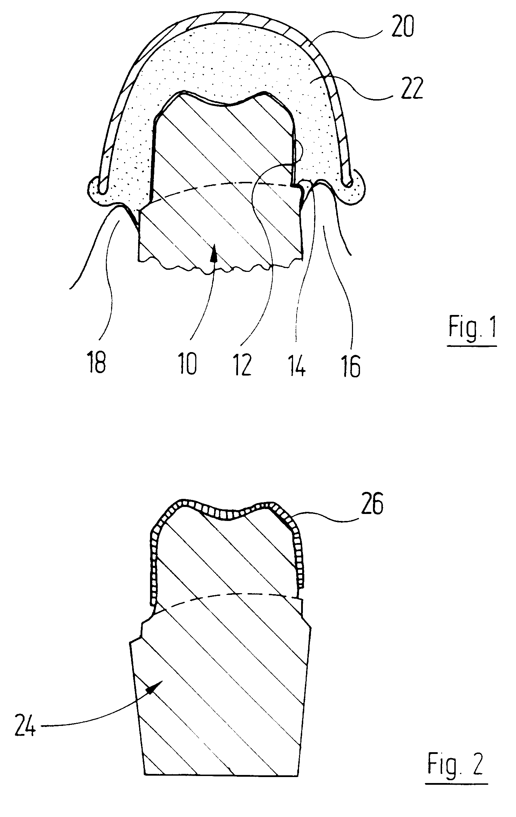

In Figur 1 ist mit 10 insgesamt ein präparierter Zahnstumpf bezeichnet. Die Präparationsfläche 12 endet in einer gebogenen, in Umfangsrichtung verlaufenden Schulterfläche 14, welche im Bereich der Zahnfleischpapillen 16, 18 liegt. Zur Herstellung dieser Vollkrone wird vom oberen Abschnitt des Zahnstumpfes ein Negativabdruck hergestellt. Hierzu findet ein Abformlöffel 20 Verwendung, der mit einem plastischen Abformmaterial 22 gefüllt ist.

Der durch das erstarrte Abformmaterial gebildete Negativabdruck wird anschließend ausgegossen, beispielsweise mit Gips. Das so erhaltene Positivmodell des Zahnstumpfes 10 wird in üblicher Weise aufbereitet (Sägeschnittsegmentmodell) und zusammen mit einem Gegenkiefermodell in einen Artikulator eingebracht.

Figur 2 zeigt das so erhaltene Positivmodell 24. Auf ihm wird eine Schutzschicht-Platzhalterschicht 26 aufgebracht, die der Kontur der Präparationsfläche 12 folgt und beim betrachteten Ausführungsbeispiel durchgehend gleiche Stärke hat. Die Platzhalterschicht 26 endet kurz über der Schulterfläche 14.

Anstelle einer gleichbleibend dicken Platzhalterschicht 26 kann man auch eine Platzhalterschicht aufbringen, deren Dicke gemäß der Beanspruchung der verschiedenen Oberflächenbereiche variiert: über Oberflächenbereichen, die stärkere Beanspruchung erfahren, ist die Platzhalterschicht 26 dicker als in weniger beanspruchten Oberflächenbereichen.

Die Platzhalterschicht 26 kann aus Wachs, einem Kunststoffmaterial oder aus einem anderen Material bestehen, welches sich später vom Positivmodell 24 mechanisch durch physikalische Einwirkung oder durch chemische Einwirkung ablösen läßt. Wahlweise kann man vom Positivmodell 24 eine Doublette herstellen, und dann Positivmodell oder Doublette mit einer nicht ablösbaren Platzhalterschicht versehen, wobei dann für später durchzuführende Herstellungschritte das von der Platzhalterschicht freie Modell verwendet wird.

Allgemein entspricht die Platzhalterschicht 26 in Form, Schichtstärke, Lokalisisation und Ausdehnung einer Schutzschicht, die später auf der Innenfläche einer keramischen Einzelkrone vorliegen soll. In der Regel erstreckt sich die Platzhalterschicht 26 über die gesamte Präparationsfläche 12, wobei ihre Dicke im Hinblick auf die spezielle individuelle Form der zu erstellenden Krone und die spätere Funktionsbelastung der Krone abgestimmt ist. Für durchschnittliche Front- oder Seitenzahnkronen ist eine Dicke der Platzhalterschicht 26 von etwa 0,3 mm geeignet. Für kaufunktionell stark beanspruchte Seitenzahnkronen, z.B. bei Patienten mit bilateral balancierter Okklusion, oder für Brückeranker bildende Kronen haben sich Schichtstärken im Bereich von 0,6 mm bewährt.

Vorzugsweise bleibt der unmittelbar der Schulterfläche 14 benachbarte Bereich der Präparationsfläche 12 von der Platzhalterschicht 26 frei, wie in Figur 2 gezeigt, oder man sieht dort eine Platzhalterschicht verminderter Dicke vor (etwa 0,1 mm bis 0,3 mm).

Das Aufbringen der Platzhalterschicht 26 auf den Positivmodel 24 kann bei Verwendung von Wachsschichten beispielsweise durch Tauchverfahren und/oder Modellieren des Wachses erfolgen. Stattdessen kann man auch adaptierte Tiefziehfolien oder ablösbare Lacke verwenden. In weiterer Abwandlung kann man die Platzhalterschicht 26 aus mehreren Teilschichten zusammensetzen, die aus einer oder mehreren der vorgenannten Schichtarten zusammengesetzt sind. Wichtig ist, daß die Platzhalterschicht 26 einerseits ausreichend stabil ist, damit von dem die Platzhalterschicht tragenden Positivmodell ein Abdruck gemacht werden kann, andererseits die Platzhalterschicht 26 ohne Beschädigung des Positivmodelles 24 von letzterem entfernt werden kann.

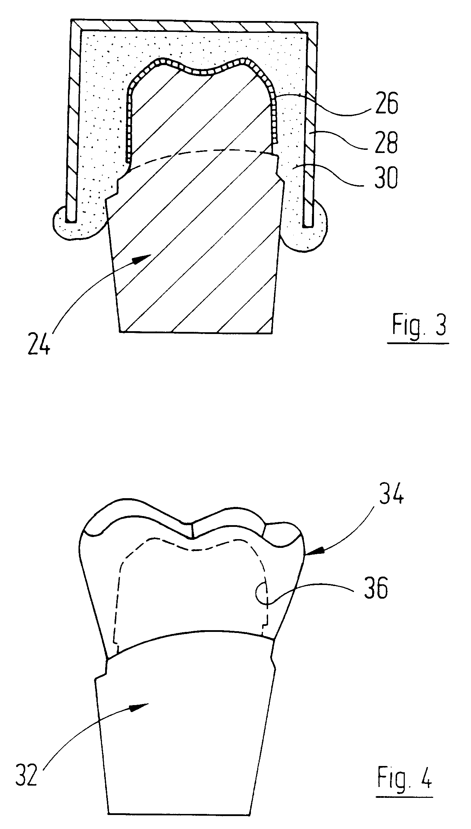

Von dem die Platzhalterschicht 26 tragenden Positivmodell wird anschließend ein Negativabdruck gemacht, wozu (vergleiche Figur 3) ein Abformbecher 28 verwendet wird, der mit einem Abformmaterial 30 gefüllt ist, z.B. einem additionsvernetzten Silikon, z.B. Polysiloxan.

Der so erhaltene Negativabdruck wird mit einer Formmasse ausgegossen, die Gips oder bevorzugt ein feuerfestes Material sein kann.

In Figur 4 ist ein so erhaltenes Arbeitsmodell 32 wiedergegeben, welches das Positivmodell des die Platzhalterschicht 26 tragenden Positivmodelles 24 darstellt.

Das Arbeitsmodell 32 wird in der gleichen Stellung wie das Positivmodell 24 im Sägeschnittsegmentmodell orientiert, wobei ein zuvor angefertigter Justierschlüssel verwendet wird, der beispielsweise aus Silikon hergestellt ist. Der Justierschlüssel bezieht sich dabei auf Oberflächen des Positivmodelles 24, welche nicht mit der Platzhalterschicht 26 versehen sind.

Über dem Arbeitsmodell 32 wird nun Keramikmaterial (z.B. ein zur zahntechnischen Verarbeitung geeignetes Feldspatporzellan für Formsintertechnik) aufgebaut welches später die Zahnkrone bilden soll. Dieses Aufbauen erfolgt mit den dem Zahntechniker bekannten Verfahren, wobei die Gestaltung der Krone individuell unter Berücksichtigung der benachbarten Zähne und insbesondere des im Gegenkiefer gegenüberliegenden antagonistischen Zahnes erfolgt.

Üblicherweise wird die Zahnkrone 34 in mehreren Schichten aufgebaut und gebrannt, funktionell an die Artikulatorsituation adjustiert und poliert. Anschließend wird die Zahnkrone 34 vom Arbeitsmodell 32 abgenommen und sorgfältig von Rückständen (z.B. an Material, aus welchem das Arbeitsmodell 32 hergestellt ist) gereinigt. Die Platzhalterschicht wird vorsichtig vom Modellstumpf 24 entfernt.

Die durch die Platzhalterschicht 26 bedingte nur lockere Passung der Zahnkrone 34 auf dem Positivmodell 24 wird zusammen mit der Fehlerfreiheit der Gestaltung der funktionellen Kronenoberflächen im Artikulator geprüft. Falls gewünscht, kann auch eine Kontrolle z.B. der Passung im Randbereich oder der Farbgestaltung durch Einprobe der Zahnkrone am Patienten erfolgen. Gegebenenfalls können Korrekturen vorgenommen werden.

Falls bei der Übertragung der Krone auf den Zahnstumpf des Patienten in diesem Stadium Ungenauigkeiten festgestellt werden, welche die Passung beeinträchtigen, kann die Krone mit konventionellem Abdruckmaterial ausgefüllt werden und auf den Zahnstumpf des Patienten reponiert werden. In diesem Fall dient die Krone (oder Brücke) als invidueller Abdrucklöffel, was die Abformgenauigkeit gegenüber dem ursprünglichen Gesamtabdruck vom gesamten Kiefer des Patienten erhöht. Es wird danach dann ein neuer Modellstumpf gefertigt, welcher dem weiteren Verfahren zugrunde gelegt wird, insbesondere einer späteren Einbringung einer Schutzschicht, wie weiter unten noch genauer beschrieben wird.

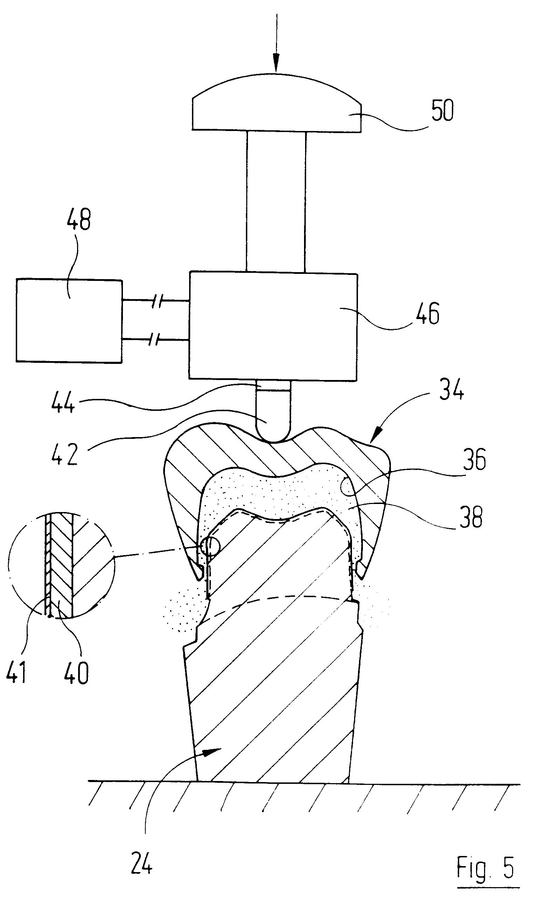

Aufgrund des Präparationsflächen-Übermaßes des Arbeitsmodelles 32 begrenzt die in Figur 5 mit 36 bezeichnete Innenfläche der Zahnkrone 34 zusammen mit der Außenfläche des Positivmodelles 24 einen Hohlraum, welcher in der Geometrie der Platzhalterschicht 26 entspricht. Dieser Hohlraum wird erfindungsgemäß mit einem Schutzschichtmaterial ausgefüllt, welches einer Rißbildung in der Keramik sowie einem Weiterwachsen etwa vorhandener Risse in der Keramik entgegenwirkt.

Das Aufbringen der Schutzschicht auf die gebrannte keramische Zahnkrone 34 kann folgendermaßen durchgeführt werden:
Auf das Positivmodell 24 wird eine Zement-Platzhalterschicht 40 aufgetragen. Deren Dicke wird entsprechend der Dicke des später zum Befestigen der Zahnkrone auf den Zahnstumpf 10 verwendeten Fügemateriales (Zement) gewählt, in der Praxis etwa 10 bis 20 µm.

Die mit einer späteren Schutzschicht (vergleiche unten) in Kontakt kommenden Oberflächen des Modellstumpfes 24 werden vorteilhafterweise zusätzlich mit einer Trennmittelschicht 41 versehen. In besonderen Fällen kann eine solche entfallen, wenn die Zement-Platzhalterschicht selbst als Trennmittelschicht wirken kann.

Geeignete Trennmittel stehen als Silikon- oder Alginat-Lösungen zur Verfügung.

Die mit der Schutzschicht zu versehende Innenfläche 36 der Zahnkrone 34 wird zunächst mit Alkohol entfettet. Je nach Art und Zusammensetzung des keramischen Werkstoffes der Zahnkrone 34 wird dann die Innenfläche 36 zur Verbesserung des Verbundes mit der später aufzubringenden Schutzschicht durch vorsichtiges Abstrahlen mit feinen Aluminiumoxidpartikeln gereinigt und ggf. mechanisch angerauht und/ oder z.B. mit Flußsäure angeätzt. Zusätzlich oder stattdessen kann eine oberflächliche Silikatanreicherung erfolgen, z. B. durch tribochemische Beschichtung.

Bei Einsatz von konventionellem Dentalporzellan zur Herstellung der Zahnkrone, z. B. feldspatkeramischen oder glaskeramischen Massen, wird die Innenfläche der Zahnkrone zunächst mit Aluminiumoxidpartikeln (bevorzugte mittlere Korngröße etwa 25 µm) gestrahlt und anschließend geätzt, z.B. mit Bifluoridlösung oder Flußsäurelösung.

Nach sorgfältiger Entfernung von Resten des Ätzmittels sowie gelöster Präzipitate z.B. im Wasserspray, oder nach Entfernen von Rückständen von Strahlmittel nach tribochemischer Oberflächenbeschichtung, z.B. im Luftstrom und nach anschließender Trocknung, wird die zu beschichtende Innenfläche 36 in herkömmlicher Weise z.B. durch Applikation einer Organosilanlösung silanisiert und ist dann bereit zum Aufbringen der Schutzschicht.

Das Aufbringen der Schutzschicht erfolgt vor der Eingliederung der Zahnkrone in den Mund des Patienten, also unabhängig vom Verbinden der Zahnkrone mit dem Zahnstumpf unter Verwendung eines Fügemateriales wie Zement. Die wie oben beschrieben vorbereitete Zahnkrone mit silanisierter Innenfläche wird mit einem ausreichenden Volumen an plastischem Schutzschichtmaterial gefüllt. Stattdessen kann das Schutzschichtmaterial als zähplastische Masse, Paste, formbare Folie, plastifizierbare Tablette oder verformbares Prepreg auf das Positivmodell 24 aufgebracht werden. Im letzten Falle können auf das Positivmodell 24 auch nacheinander unterschiedliche Teilschichten der Schutzschicht aufgebracht werden.

In Abwandlung kann man auch einen Teil des Schutzschichtmateriales auf den Modellstumpf aufbringen und einen weiteren Teil des Schutzschichtmateriales auf die Innenfläche der Zahnkrone aufbringen.

Anschließend wird die Zahnkrone 34 vollständig auf das Positivmodell 24 aufgeschoben. Dieses Aufschieben erfolgt bei hohe Viskosität aufweisenden Schutzschichtmaterialien, insbesondere thixotropen Schutzschichtmaterialien unter zusätzlicher Unterstützung durch oszillierende, vorzugsweise ultraschallaktivierte Werkzeuge. Hierzu greift an der Oberseite der Zahnkrone 34, wie in Figur 5 gezeigt, ein Drückinstrument 42 an, welches mit dem Abtriebsteil 44 eines Ultraschallgenerators 46 verbunden ist. Letzterer wird von einem Versorgungsgerät 48 her erregt und hat einen zum Ausüben axialen Druckes (vergleiche Pfeil in Figur 5) geeigneten Handgriff 50.

In Abwandlung kann man zum Aufschieben der Zahnkrone 34 die Viskosität des Schutzschichtmateriales auch durch Wärmeeinwirkung vorübergehend heruntersetzen, wobei vorzugsweise die Zahnkrone 34 oder das Positivmodell 24 vorab auf geeignete Temperatur aufgeheizt werden. Gegebenenfalls kann das Aufschieben der Zahnkrone 34 auf das Positivmodell 24 auch in Teilschritten erfolgen, zwischen denen die Zahnkrone jeweils wieder abgenommen wird, so daß das Schutzschichtmaterial direkt nochmals erwärmt werden kann. Das letztgenannte Vorgehen findet insbesondere bei der Verarbeitung thermoplastischer Schutzschichtmaterialien Verwendung.

Generell ist das verwendete Volumen an Schutzschichtmaterial so groß, daß beim vollständigen Aufdrücken der Zahnkrone 34 auf das Positivmodell 24 überschüssiges Material ausgepreßt wird. Nach sorgfältiger Entfernung des gesamten ausgepreßten Materiales können die Passung der Zahnkrone, die Kontaktpunkte mit den benachbarten Zähnen sowie die funktionellen Okklusions- und und Artikulationskontakte geprüft werden.

Bei Verwendung von acrylathaltigen Schutzschichten ist durch Applikation von Glyceringel, vorzugsweise auf die dem Kronenrandbereich entsprechende Randbereiche der noch nicht erhärteten Schutzschicht gewährleistet, daß sich keine sauerstoffinduzierten oberflächlichen Polymerisationsinhibitionsschichten bilden. Man erhält so reproduzierbare mechanische Eigenschaften der Schutzschicht bis in deren Randbereiche.

Das Polymerisieren des Schutzschichtmateriales erfolgt beispielsweise durch Bestrahlen mit Licht geeigneter Wellenlänge und/oder Einwirkung von Wärme oder auch autokatalytisch. Bei thermoplastischen Schutzschichtmaterialien erfolgt die Verfestigung der Schutzschicht durch Abkühlung.

Durch Anwendung der oben beschriebenen Technik lassen sich auch dünn ausfließende Ränder von Zahnkronen oder anderen Zahnrestaurationsteilen reproduzierbar rekontruieren, was bei herkömmlicher Dentalkeramiktechnik nicht möglich ist, da bei dünn ausfließenden Rändern auf Grund des Sprödbruchverhaltens der Keramik Randausbrüche auftreten.

Beim Aufsetzen der Zahnkrone 34 auf das Positivmodell 24 wird vorzugsweise ein oft als Schlüssel bezeichnetes Justier-Hilfsmittel verwendet, um sicherzustellen, daß die Achsrichtung und die Winkellage der Zahnkrone bezüglich der Zahnstumpfachse korrekt sind. Ein solcher Schlüssel kann entbehrlich sein, wo die Schulterfläche 14 oder ein anderer von der Platzhalterschicht 26 nicht bedeckter Oberflächenabschnitt schon ausreichend charakteristisch ausgebildet ist. Üblicherweise wird ein solcher Schlüssel aus Gips oder Hartsilikon hergestellt und weist Oberflächeneindrücke der Krone und des Modellstumpfes auf. Zum Aufsetzen der Krone wird dieser Schlüssel durch den z.B. mit der Labialfläche des Positivmodelles verbunden und die Krone wird solang auf dem Positivmodell (unter Verdrängung des schon eingebrachten Schutzschichtmateriales) verdreht, gekippt und verschoben, bis ihre Oberflächen exakt in die komplementären Oberflächeneindrücke des Schlüssels eingreifen.

Anstelle eines Schlüssels kann man auch kleine Positionierkerben und entsprechende Positionierrippen auf dem Positivmodell und der Zahnkrone verwenden, wobei die von der Zahnkrone getragenen Positioniermittel in einem letzten Schritt der Kronenherstellung entfernt werden.

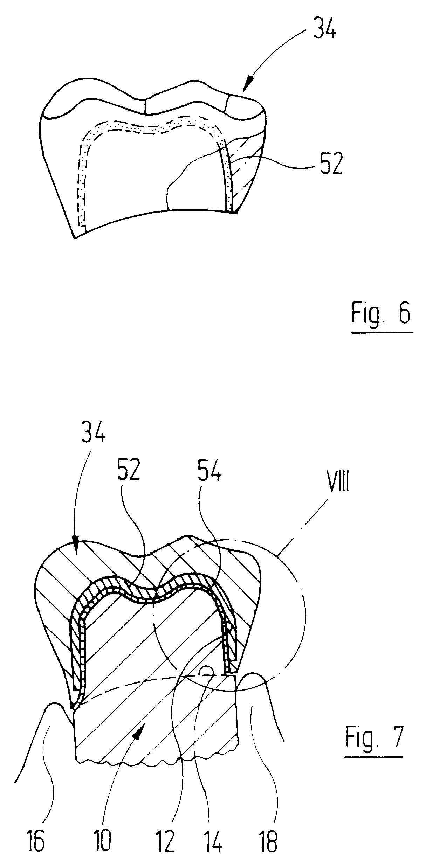

Nach vollständiger Erhärtung der Schutzschicht wird der Randbereich der Zahnkrone ausgearbeitet und poliert. Nach Abnahme der fertiggestellten Zahnkrone 34 vom Positivmodell 24 und Reinigung der Zahnkrone von Trennmittelrückständen ist die Zahnkrone fertig zur Eingliederung durch den Zahnarzt. Dieser Zustand ist in Figur 6 wiedergegeben. Man erkennt, daß sich die erfindungsgemaße Zahnkrone von herkömmlichen keramischen Zahnkronen durch die bei der Innenfläche 36 vorgesehene Schutzschicht 52 unterschiedet, deren freie Oberfläche exakt komplementär zum Positivmodell 24 und damit zum Zahnstumpf 10 ist.

Das Befestigen der Zahnkrone 34 am Zahnstumpf 10 erfolgt bevorzugt durch herkömmliche Zementierung unter Verwendung eines Zinkoxid-Phosphatzementes oder eines Glasionomerzementes oder eines Kompomers oder eines ähnlichen Zementes. Das Befestigen der Zahnkrone kann auch mit einem polymerhaltigen Befestigungskomposit erfolgen, wobei hier die sonst bei keramischen Zahnrestaurationsteilen notwendige zeitaufwendige Ätzung und Silanisierung der Kroneninnenfläche entfallen kann. Die herkömmliche Zementierung ist jedoch deshalb vorteilhafter, weil das Eingliedern wesentlich schneller und einfacher durchzuführen ist und Rückstände an Fügematerial zuverlässig vermieden werden können.

Figur 7 zeigt einen Schnitt durch die eingegliederte Zahnkrone 34 und den Zahnstumpf 10 zusammen mit der zwischen der Zahnkrone und der Präparationsfläche 12 liegenden Zementschicht 54.

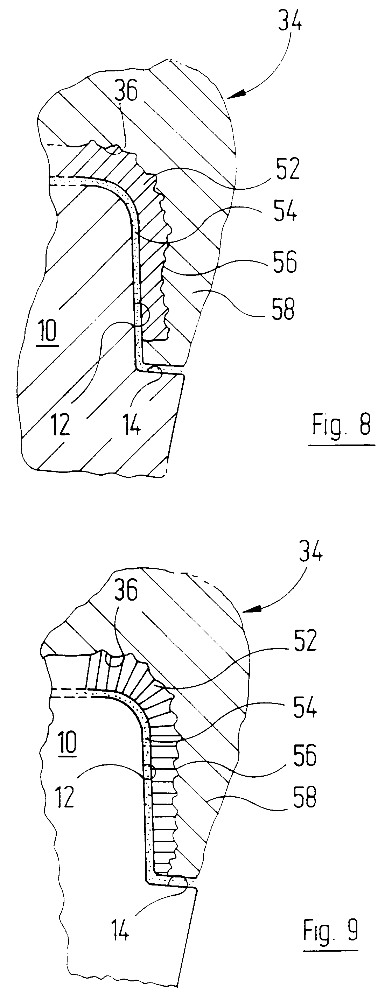

Figur 8 zeigt einen vergrößerten Ausschnitt aus Figur 7. Man erkennt, daß die Außenfläche 56 der Schutzschicht 52 und die Innenfläche des Keramikvolumens 58 der Zahnkrone 34 zueinander komplementäre unregelmäßige Rauhigkeiten aufweisen, so daß sich das Schutzschichtmaterial und das Keramikmaterial 58 lokal gut miteinander verhaken. Dieses Verhaken ist im Hinblick auf das Ausüben von tangentialer Druckspannung von der Schutzschicht auf das Keramikvolumen von Vorteil. Der Verzahnung von Schutzschichtmaterial und Keramikmaterial ist auch im Hinblick auf eine Vergrößerung der Berührfläche zwischen diesen beiden Materialien von Vorteil.

Bei der Zahnkrone 34, deren Herstellung obenstehend unter Bezugnahme auf die Figuren 1 bis 8 erläutert wurde, erstreckt sich die Schutzschicht 52 bis in die Nähe der Schulterfläche 14, jedoch nicht bis zu dieser.

Gemäß der Variante nach Figur 9 ist die Schutzschicht 52 der Zahnkrone 34 bis an die Schulterfläche 14 gezogen.

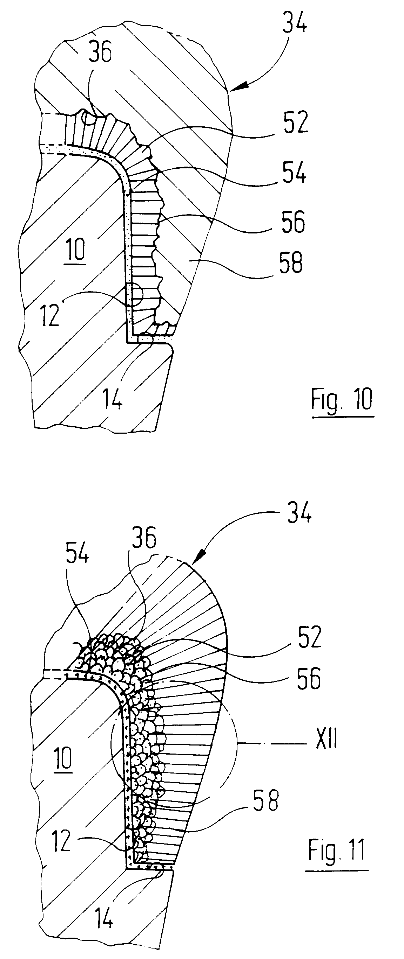

Beim Ausführungsbeispiel nach Figur 10 ist das untere Ende der Schutzschicht 52 parallel zur Schulterfläche 14 bis zur Außenseite der Zahnkrone 34 gezogen.

Beim Ausführungsbeispiel nach Figur 11 besteht die Schutzschicht 52 ihrerseits aus einem keramischen Schaummaterial, welches vor der Herstellung des Zahnrestaurationsteiles anstelle oder zusätzlich zu einer Platzhalterschicht auf den Modellstumpf oder einen nach Abformung dieses Modellstumpfes erstellten weiteren Modellstumpf vorzugsweise aus feuerfester Masse aufgetragen ist. Es kann sich hierbei um anorganisches Material, z.B. Keramik, insbesondere Aluminiumoxidkeramik, Zirkonoxidkeramik, Siliciumkarbidkeramik usw. handeln. Der keramische Schaum liegt bevorzugt als Konfektionsbauteil vor, welches durch z.B. manuelles Beschleifen oder durch Eindrücken des Modellstumpfes in den Schaum unter kontrolliertem Zerbrechen der Trabekel an den Modellstumpf angepaßt wird. Ausdehnung und Dicke werden durch manuelle Bearbeitung, z.B. mit Schnitzhandinstrumenten oder mit rotierenden Diamantinstrumenten gestaltet. Alternativ kann eine maschinelle Bearbeitung erfolgen, wie weiter unten beschrieben.

Danach wird die Restauration unter wenigstens teilweise Infiltration der Keramikmasse, welche die Krone bildet, in die Schaumstruktur gefertigt. Die Schaumstruktur verbleibt als Bestandteil der definitiven Restauration und wird bevorzugt auf der stumpfzugwandten Seite zusätzlich mit Schutzschichtmaterial infiltriert. Verwendet man durch Pyrolyse erzeugte Schäume oder Metallschäume, kann auch der Schaum zuerst (vor Brand der Krone) oder danach (in die fertige Krone hinein) hergestellt werden.

Man kann eine solche Schutzschicht auch dadurch herstellen, daß man auf die oben beschriebene Weise in den durch die Zahnkrone 34 und das Positivmodell 24 begrenzten Formraum einen wenigstens teilweise siliziumorganischen Werkstoff einbringt, der nach Pyrolyse ein wenigstens partiell keramisches Schaummaterial ergibt. Es kann sich hierbei z.B. um Polysilazane, Polysiloxane bzw. oder aus ihnen hergestellte Komposits handeln, die durch Pyrolyse keramisieren.

Schließlich kann man die Schutzschicht auch aus Metallpulvern aufbauen, z.B. Titan und Goldpulvern, die in Form einer Paste eingebracht werden, wie oben beschrieben, und dann durch Wärmeeinwirkung dicht oder unter Ausbildung von Porositäten, z.B. als Schaumstrukturen zusammengesintert werden, oder durch Sputtern oder ein sonstiges bekanntes Verfahren zum Aufbringen von Metallschichten aufgebracht werden. Dabei kann bei hochschmelzenden Metallen gegebenenfalls ein bei niedereren Temperaturen schmelzendes metallisches oder keramisches Bindemittel zugefügt werden.

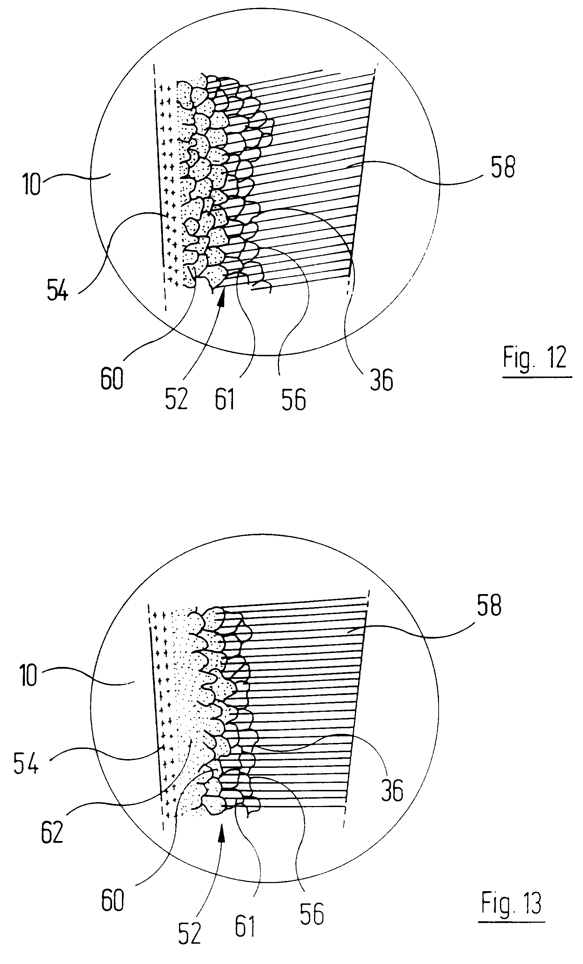

Beim Ausführungsbeispiel nach Figur 12 besteht die Schutzschicht 52 wiederum aus einem keramischen Schaummaterial, wobei eine der der Innenfläche 36 benachbarte Teilschicht 60 der offenporigen Schutzschicht mit Kunststoffmaterial infiltriert worden ist, während eine dem Keramikvolumen benachbarte Teilschicht 61 der Schutzschicht mit Keramikmaterial infiltriert wurde.

Die Herstellung eines solchen Materiales erfolgt so, daß man zunächst über dem Positivmodell die Schutzschicht 52 herstellt, aus einem Konfektionsteil herstellt oder diese wenigstens teilweise brennt, sintert oder pyrolisiert, so daß man eine offenporige keramische Schaummaterialschicht erhält, und daß über der so erhaltenen offenporigen Schutzschicht 52 dann die Zahnkrone 34 aufgebaut wird, wobei erste Anteile des Keramikmateriales, die schlickerähnliche Konsistenz haben können, in die äußeren Bereiche der Schutzschicht 52 eindringen. Alternativ kann die Schaumstruktur nach dem Anpassen vom Modellstumpf abgenommen werden und die Krone darauf aufgebaut werden, z.B. aus Feldspatkeramik, wobei auf einen feuerfesten Stumpf verzichtet wird.

Bei z.B. glaskeramischen Herstellungsrouten bzw. Kronenmaterialien wird ein Modell der Krone z.B. aus Wachs oder Kunststoff unter Einbeziehung des Schaums hergestellt, dieses in eine Negativform eingebettet und Glas bzw. Keramik nach Ausbrennen des Modellmateriales in die Muffel unter wenigstens teilweise Infiltration des Schaumes eingepreßt bzw. eingeschleudert.

Nach Brennen der Zahnkrone 34 wird dann der der Innenfläche 36 zugewandte Bereich der offenporigen Schutzschicht 52 mit Kunststoffmaterial infiltriert.

Das Ausführungsbeispiel nach Figur 13 ähnelt demjenigen nach Figur 12, nur wurde die Schutzschicht 52 auf einem unter Verwendung einer Platzhalterschicht hergestellten Arbeitsmodell aufgebaut, wie es in Figur 4 gezeigt ist. Durch Aufsetzen der so erhaltenen Zahnkrone auf das Positivmodell 24 und Füllen des zwischen Zahnkrone und Positivmodell begrenzten Formraumes mit Kunststoff erhält man dann die in Figur 13 gezeigte Struktur, bei welcher die der Innenfläche 36 benachbarte Teilschicht 60 der offenporigen Schutzschicht 52 mit Kunststoffmaterial infiltriert ist, über der so infiltrierten offenporigen Schutzschicht 52 nun aber eine weitere Teilschicht 62 liegt, die nur aus (gegebenenfalls mit Füllstoffen versehenem) Kunststoff besteht.

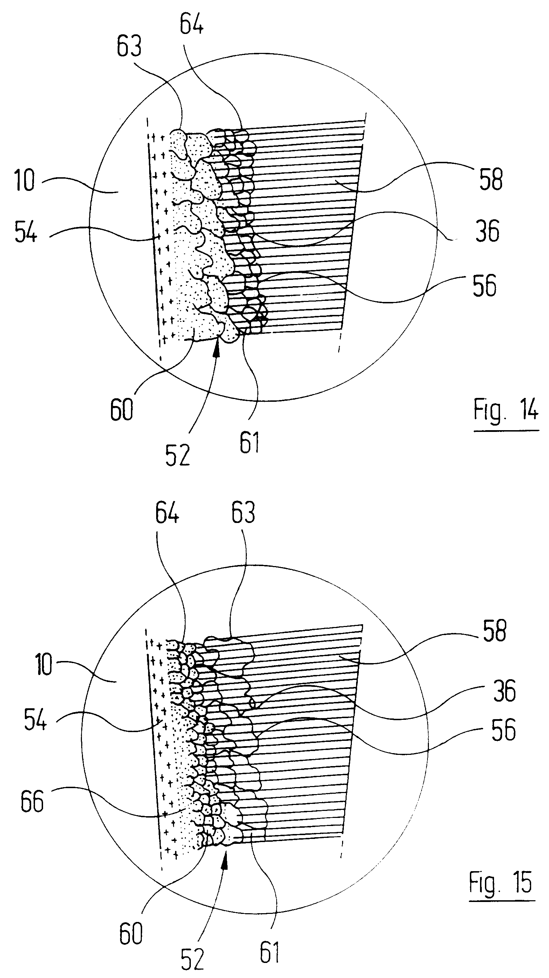

Beim Ausführungsbeispiel nach Figur 14 hat die wiederum von der einen Seite her mit Keramikmaterial, von der anderen Seite her mit Kunststoffmaterial infiltrierte offenporige Schutzschicht 52 eine anisotrope Porenstruktur. In der Nachbarschaft der Innenfläche 36 hat man größere Poren 63, in den dem Keramikmaterialvolumen benachbarten Bereichen kleinere Poren 64.

Die Herstellung oxidkeramischer Schäume erfolgt so, daß Schaumstoffmaterial zusammengepreßt wird, in Schlickermasse eingetaucht wird und im eingetauchten Zustand der Schaumstoff expandiert. Dabei wird Schlicker in die Poren eingesaugt. Anschließend kann durch definierte Brandführung (sehr hohe Temperaturen) der Schaumstoff unter Erzeugung einer dicht gesinterten Schaumstoffstruktur verbrannt werden. Diese Schäume werden heutzutage als Filter z.B. beim Guß von Aluminium eingesetzt und sind am Markt als Konfektionsbauteile vorhanden.

Anisotrope oxidkeramische Schäume erzeugt man aus anisotropen Schaumstoffverbunden komplementärer Porenstruktur.

Derartige Schaummaterialien kann man ferner z.B. dadurch herstellen, daß man die Schutzschicht 52 aus Teilschichten aufbaut, die man durch Zusammensintern von Partikeln größeren bzw. kleineren Durchmessers erhält. Alternativ kann man Teilschichten unterschiedlicher Kunststoffmaterialien, die bei Pyrolyse Keramikschaum unterschiedlicher Porengröße ergeben, übereinander erzeugen.

Das Ausführungsbeispiel nach Figur 15 ähnelt demjenigen nach Figur 14, nur befinden sich die großen Poren 63 in der Nachbarschaft des Keramikmaterialvolumens, während die kleinen Poren 64 der Innenfläche 36 benachbart sind. Außerdem hat man in einem Teilbereich der Innenfläche wieder eine nur aus Kunststoff bestehende Teilschicht 60.

Die Verwendung von Schaummaterialien bei der Herstellung der Schutzschicht 52 bringt den Vorteil, daß man einen Teil der für die Schutzschicht gewünschten Elastizität aus der Porenstuktur der beteiligten Werkstoffe erhält und nicht über Volumeneigenschaften des Werkstoffes bereitstellen muß.

Dies gilt insbesondere unter Verwendung von im Zentrum hohlen Partikeln zur Herstellung von Schäumen, insbesondere von metallischen Hohlkugeln.

Weitere Vorteile der Verwendung von Schaumstrukturen sind
- die vergleichsweise einfache Herstellung einer Art Gerüst, auf dem bzw. in das die Restauration aufgebaut wird;
- die Herstellung einer mehrteiligen Gradientenstruktur, wobei das Schaummaterial nur wenig Volumen beansprucht (Kronen haben nur limitierte Schichtstärke);
- die individuelle mehrfarbige Gestaltung der Restauration durch die zu infiltrierende Masse, z.B. Keramik- oder Kunststoffmasse (Farbwirkung kann auch aus der Tiefe kommen, während bisher nur eine Oberflächenschicht der Zahnrestauration für die Farbe verantwortlich war).

Ein anisotropes Schaummaterial mit großen Poren und kleinen Poren ist vorteilhaft, weil man so die für das jeweils zu infiltrierende Material günstigsten Bedingungen vorgeben kann. Dabei kann man durch die unterschiedliche Porengröße gewährleisten, daß das schlechter infiltrierende Material die großen Poren sicher und im wesentlichen vollständig ausfüllt und die einzelnen Schaumtrabekeln sicher rundum benetzt, während die kleineren Poren, die mit einem anderen Material zu infiltrieren sind, zunächst ungefüllt bleiben. Diese können dann später mit diesem anderen Material, welches in seiner Viskosität oder seinem Benetzungsverhalten gegenüber den Trabekeln anders eingestellt ist, gegebenenfalls unter Zuhilfenahme spezieller Benetzungsmittel und ebenfalls gegebenenfalls unter zusätzlicher Unterstützung durch Druck, Wärme oder Ultraschall gefüllt werden.

Ein weiterer Vorteil der Verwendung von Schaummaterialien für die Schutzschicht ist der, daß man auf ein Ätzen der Innenfläche der Keramikkrone verzichten kann.

Besonders geeignete Schaummaterialien sind Schäume aus Aluminiumoxidkeramik. Dabei sind Porendichten im Bereich von 30 Poren pro Inch bis 120 Poren pro Inch (12-48 Poren/cm), vorzugsweise von 50 PPI bis 90 PPI (20-35 Poren/cm) und nochmals bevorzugt im Bereich von 65 PPI bis 80 PPI (25-32 Poren/cm) gut geeignet.

Bei den oben beschriebenen Ausführungsbeispielen bestand die Schutzschicht zumindest teilweise zusätzlich aus Kunststoffmaterial.

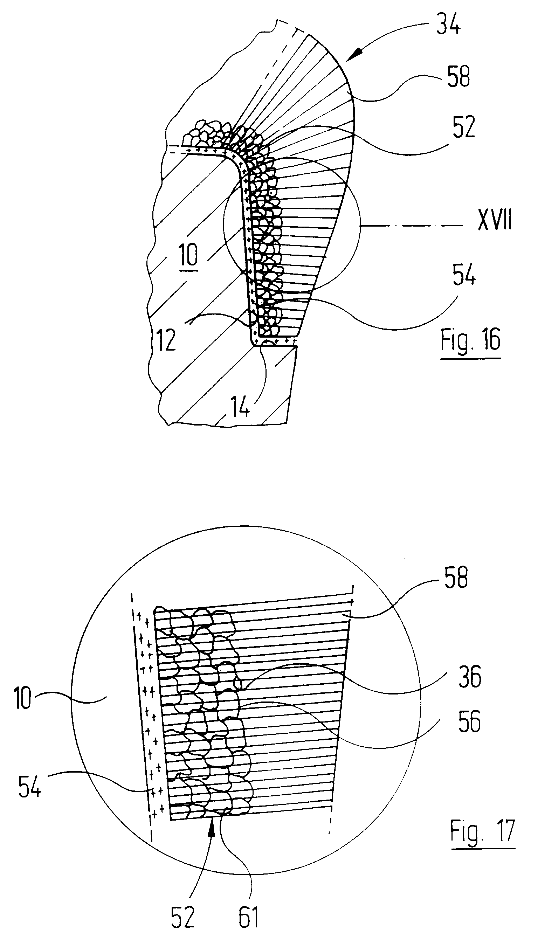

Beim Ausführungsbeispiel nach Figur 16 und 17 besteht die Schutzschicht aus Schaummaterial, welches bis in die Nachbarschaft der Innenfläche 36 mit dem Keramikmaterial infiltriert ist, welches auch das Keramikvolumen 58 bildet. Wie oben schon beschrieben, kann das Schaummaterial wiederum ein keramisches Schaummaterial oder ein metallisches Schaummaterial sein.

Alternativ zu den Beispielen nach den Figuren 16 und 17 kann man auch Schaumstrukturen verwenden, wobei das Schaummaterial, z.B. metallisches Schaummaterial oder siliziumorganisches Schaummaterial, teilweise pyrolytisch wenigstens teilweise bei der Herstellung und bei dem Brand der Krone bzw. bei einem nachträglichen Tempern derselben umgesetzt oder verbrannt werden oder einer Volumenschwindung unterliegen. Auf diese Weise verbleibt ein Negativ des Reliefs der Schaumstruktur in der Zahnkrone.

Weist die ohne Schutzschicht versehene keramische Restauration auf der stumpf zugewandten Seite wenigsten teilweise offene Porositäten auf oder können solche beispielsweise durch physikalische Bearbeitung, wie z.B. durch Sandstrahlen und/oder durch chemische Behandlung, wie z.B. durch Applikation von sauren oder basischen Lösungen, und/oder elektrochemische Behandlung, vorzugsweise durch Herauslösen von in diese Grenzflächen eingebrachten, z.B. mit metallischen, organischen oder anorganischen Bestandteilen oder metallischen oder keramischen Schaumstrukturen, bis in eine Tiefe, welche dem Minimum der ansonsten üblichen Stärke der Schutzschicht entspricht, erzeugt werden, so kann auf die Applikation einer Platzhalterschicht vor der Herstellung dieser keramischen Restauration verzichtet werden. Die Schutzschicht entsteht in diesen Fällen durch Infiltration dieser vorzugsweise miteinander kommunizierenden Porositäten. Die Grenzflächen können zur Verbesserung der Anhaftung der Schutzschicht vor der Infiltration konditioniert, insbesondere silanisiert werden. Für diesen Fall haben sich als infiltrierbare Massen vorzugsweise weniger hoch gefüllte Kunststoffe bewährt. Durch die vergleichsweise große Oberfläche der Kontaktzone zwischen der keramischen Phase und der in die poröse Grenzfläche einpenetrierten bzw. infiltrierten Schicht kann deren Schichtstärke im Vergleich zu einer auf die zu schützende Stelle des Restaurationsteiles aufgebrachten Schicht bei gleicher Funktionssicherheit verringert werden. Bei einem Porenanteil von 50% bezogen auf das Gesamtvolumen der zu infiltrierenden Grenzfläche genügt in aller Regel eine Infiltrationstiefe von 0,1 bis 0,2 mm.

Es ist auch möglich, eine Kombination einer in eine poröse keramische Grenzfläche infiltierten Schutzschicht mit einer auf die Grenzfläche aufgetragenen Schicht möglich. Das Anbringen dieser Schichten kann in einem einzigen gemeinsamen oder in mehreren Arbeitsgängen erfolgen, vorzugsweise jeweils unter vollständiger Reposition der Restauration auf den Modellstumpf.

Eine weitere Möglichkeit der Herstellung von erfindungsgemäßen Restaurationen besteht darin, daß die auf dem Modellstumpf oder auf einem form- und dimensionsgleichen Duplikatmodell von diesem hergestellte Platzhalterschicht aus einem Material besteht, das unter den zur Herstellung der keramischen Restaurations erforderlichen Brenntemperaturen formstabil und dimensionstreu ist. Der Wärmeausdehnungskoeffizient eines solchen Materials sollte dem der gewählten keramischen Massen entsprechen, die Platzhalterschicht ohne Verzüge vom Modellstumpf ablösbar sein und den manuellen Kräften im Zuge des Aufsinterns des Verblendmassen Stand halten. Die keramische Restauration kann dann direkt auf dieser, für Zahnkronen etwa käppchenartig ausgebildeten Platzhalterschicht hergestellt werden. An Materialien für diese Platzhalterschicht eignen sich beispielsweise Folien aus Platin bzw. Platinlegierungen. Auch eignen sich z.B. aus siliziumorganischen Werkstoffvorstufen hergestellte Platzhalterschichten, welche gleichzeitig mit dem Aufbrennen der ersten Dentalkeramikschicht oder in einem vorhergehenden Randzyklus pyrolytisch keramisiert werden.

Einen ähnlichen Effekt kann man auch dadurch erhalten, daß man einen Teil der Trabekel selektiv entfernt, z.B. durch Ätzen.

Der Schaum wird dann z.B. unter Einbringung von Polymeren, Metallen oder anorganischen Werkstoffen oder Verbundwerkstoffen in dieses Negativrelief konstruiert, welches die fertige Krone aufweist.

Schaumstrukturen können auch so erzeugt werden, daß man normale Dentalkeramikmasse auf einem feuerfesten Modellstumpf brennt, wobei das Stumpfmaterial bei höherer Temperatur Gasblasen, z.B. Stickstoffblasen freigibt (Stickstoff aus Harnstoff o.ä.). Diese Gasblasen bilden in der Randzone der Krone offene Porositäten, die mit Kunststoff, Metall oder Keramik infiltriert werden können und mit den letztgenannten Materialien einen Verbundwerkstoff bilden. Die Schaumstruktur kann auch, soweit sie pyrolysierbar ist, wenigstens teilweise keramisiert werden.

In den Figuren 18 und 19 ist eine andere Variante der Herstellung des Raumes zur späteren Aufnahme einer Schutzschicht dargestellt: Über dem Positivmodell 24 ist eine Platzhalterschicht 26 aus Wachs oder Kunststoff angebracht. Über diese ist die Zahnkrone 34 aufgebaut. Nach Abnehmen der geformten Zahnkrone und Entfernung der Platzhalterschicht wird diese in eine feuerfeste Einbettmasse eingebettet. Das Wachs- oder Kunststoffmaterial fließt bzw. brennt während der thermischen Behandlung aus und die Restauration wird unter Einpressen bzw. Einschleudern von Glaskeramik oder Keramik gefertigt und ggf. zur Ausbildung bzw. zum Wachstum von volumeninhärenten Kristallen anschließend getempert.

Beim Ausführungsbeispiel nach Figur 19 ist schon das Positivmodell 24 nicht aus einem feuerfesten Material hergestellt, und die Platzhalterschicht 26 besteht aus einem feuerfesten Material, z.B. Platin. Über dieser Platzhalterschicht 26 ist das Keramikmaterial der Zahnkrone 34 aufgebaut. Die Zahnkrone kann ohne Positivmodell 24 nur auf der Platzhalterschicht 26 gebrannt werden. Nach dem Brennen wird die Platzhalterschicht 26 mechanisch entfernt. Dann wird auf die Zahnkrone 34 die Schutzschicht 52 aufgebracht, wie obenstehend unter Bezugnahme auf Figur 5 beschrieben.

Die Figuren 20 und 21 zeigen eine weitere Möglichkeit der Herstellung eines Übermaß-Arbeitsmodelles 32.

Im Abformbecher 28 ist ein Abformmaterial 30 enthalten, welches bei Aushärtung im Volumen schwindet. Unter Verwendung dieses Materiales wird vom Positivmodell 24 ein Negativabdruck gemacht. Figur 21 zeigt die Verhältnisse nach dem Aushärten des Abformmateriales 30. Man erkennt zwischen der Innenfläche des Negativabdruckes und der Außenfläche des Positivmodelles 24 einen Zwischenraum 66, welcher die Geometrie einer später auf der Krone zu erzeugenden Schutzschicht vorgibt. Der Abformbecher ist durch Löcher oder durch Beschichten so gestaltet, daß das Material aufschrumpft.

Der Abformbecher 28 ist so gestaltet und dimensioniert, daß die durch Schwindung erhaltene Dicke des Zwischenraumes 66 in den den Präparationsgrenzen benachbarten Abschnitten kleiner ist als in denjenigen Bereichen, die der okklusalen Oberfläche des Positivmodelles 24 benachbart sind.

Gießt man den in Figur 21 gezeigten Negativabdruck mit einer feuerfesten Doubliermasse aus, so erhält man das in Figur 22 gezeigte Arbeitsmodell 32, welches gegenüber dem Positivmodell 24, dessen Konturen gestrichelt eingetragen sind, dem Zwischenraum 66 entsprechendes Übermaß aufweist. Das Arbeitsmodell 32 nach Figur 22 kann dann ähnlich weiterverwendet werden wie das in Figur 4 gezeigte Arbeitsmodell.

Stattdessen kann man einen dimensionstreuen Negativabdruck herstellen und mit einer expandierenden Modellmasse das Arbeitsmodell 32 herstellen. Derartige Modellmassen sind z.B. Materialien, welche durch Kristallisation im Rahmen einer Wärmebehandlung ihr Volumen vergrößern.

Eine weitere Möglichkeit der Herstellung eines Arbeitsmodelles, die in der Zeichnung nicht wiedergegeben ist, besteht darin, ähnlich wie in Figur 3, jedoch ohne Vorsehen einer Platzhalterschicht, einen Negativabdruck des Positivmodelles 24 zu machen. Ausgehend von diesem Negativabdruck wird dann unter Verwendung einer speziellen Modellmasse ein Arbeitsmodell hergestellt. Diese Modellmasse hat die Eigenschaft, daß sie zunächst im Formschluß mit dem Negativabdruck so weit fest werden kann, daß sie handhabbar ist und ihre Geometrie beibehält. Durch eine anschließende Wärmebehandlung, durch Kristallisation oder eine chemische Behandlung kann dann das Material des Arbeitsmodelles zum Expandieren gebracht werden. Diese Expansion kann z.B. ein restlicher Anteil eines Schäumvorganges sein. Das Übermaß-Arbeitsmodell wird dann ähnlich verwendet, wie obenstehend unter Bezugnahme auf Figur 4 ff beschrieben.

In weiterer Abwandlung der Erfindung kann man einen Teil des Übermaßes des Arbeitsmodelles dadurch erzeugen, daß man einen Negativabdruck des Positivmodelles 24 mit einer schwindenden Modellmasse herstellt, wie oben beschrieben, und in den so erhaltenen Übermaß-Negativabdruck eine Modellmasse einfüllt, die nach der Form expandiert. Auf diese Weise lassen sich besonders große Übermaße realisieren.

Im Prinzip ist es auch möglich, ein Modell des Restaurationsteils direkt auf den Zahnstumpf 10 im Munde des Patienten zu formen, zumindest was die Innenfläche 36 betrifft. In diesem Fall wird dann auf den Zahnstumpf 10 bervorzugt ein Trennmittel aufgetragen. Zur Schaffung eines Raumes für die Schutzschicht 52 kann dann von dem vorgehärteten Rohling des Restaurationsteiles spanend Material abgenommen werden. Falls gewünscht, kann man statt dieser Materialabnahme über dem Zahnstumpf 10 auch eine Platzhalterschicht 26 aufbauen und dann wie oben beschrieben verfahren. Alternativ oder zusätzlich kann man ein Übermaß-Arbeitsmodell auch so herstellen, daß man vom Negativabdruck materialchemisch oder chemomechanisch abträgt. Soll ein solcher Abtrag in unterschiedlichen Flächenbereichen des Negativabdruckes unterschiedlich erfolgen, kann man den Negativabdruck mit einem lichtempfindlichen Schutzlack überziehen, der dann in den nach der Entwicklung verbleibenden Bereichen die chemomechanische oder chemische Materialentfernung an den entsprechenden Stellen verhindert.

In solchen Fällen, in denen die Schutzschicht 52 aus Schaummaterial besteht, kann man schon die Platzhalterschicht 26 aus diesem Material herstellen und anschliessend in Verbund mit dem Keramikmaterial bringen.

Derartige Schaummaterialien sind auch in Form plastisch verformbarer Folie erhältlich, die somit durch Biegen und Pressen dem Modellstumpf angepaßt werden können.

Es sind aber auch für Schutzschichten geeignete Schaummaterialien im Handel erhältlich, insbesondere Aluminiumoxidkeramik-Schaummaterialien, die sich gut für eine spanende Bearbeitung eignen. In diesem Falle kann man dann für die Herstellung eines Platzhalterschichtteiles oder eines später in die Keramik zu integrierenden Schutzschichtteiles so vorgehen, wie nachstehend unter Bezugnahme auf Figur 23 im einzelnen erläutert wird:

Figur 23 zeigt schematisch eine Vorrichtung zum Erzeugen eines Übermaß-Arbeitsmodelles ausgehend von einem Positivmodell 24, welches auf einer Spindel 68 befestigt ist, die durch einen elektrischen Getriebemotor 70 gedreht wird. Zur Bestimmung der Spindelstellung ist ein Winkelgeber 72 vorgesehen.

Der Getriebemotor 70 sitzt auf einem Schlitten 74, der auf einer Führungsstange 76 läuft und durch eine Gewindespindel 78 angetrieben wird. Auf letztere arbeitet ein Getriebemotor 80. Zur Bestimmung der Winkelstellung der Gewindespindel 78 und damit der Axialposition des Schlittens 74 dient ein weiterer Winkelgeber 82. Der Getriebemotor 80 ist auf einer Platte 84 angeordnet, die von einem feststehenden Rahmenteil 86 der Vorrichtung getragen ist.

Mit der Umfangsfläche des Positivmodelles 24 arbeitet ein erster Linearstellungsgeber 88 zusammen, der von einem Rahmenteil 90 des Vorrichtungsrahmens getragen ist. Zur Erfassung der Stirngeometrie des Positivmodelles 24 dient ein weiterer Linearstellungsgeber 92, der auf einem Schlitten 94 angeordnet ist, der auf einer Führungsstange 96 läuft und durch eine Gewindespindel 98 bewegt wird. Auf letztere arbeitet ein Getriebemotor 100. Die Radialstellung des Linearstellungsgebers 92 bezüglich der Achse der Spindel 68 wird durch einen Winkelgeber 102 gemessen, der mit der Gewindespindel 98 zusammenarbeitet.

Die Ausgangssignale der verschiedenen Stellungsgeber und Winkelgeber sind mit Schnittstellen eines Prozeßrechners 104 gekoppelt, der aus den verschiedenen beim Abtasten der Oberfläche des Positivmodelles 24 erhaltenen Stellungssignalen ein digitales Bild der Oberfläche des Positivmodelles 24 erzeugt und dieses auf einem Massenspeicher 106, z.B einer Festplatte, ablegt. Auf letzterem sind auch die verschiedenen Programme abgelegt, nach denen der Prozeßrechner steuert, darunter zum einen ein Programm, welches zum Erzeugen des digitalen Bildes des Positivmodelles 24 dient, und zum anderen ein Programm, das eine in definierter Weise verschobene Übermaß-Kontur der Oberfläche des Positivmodelles 24 erzeugt und so ein digitales Bild einer Platzhalterschicht bzw. der Außenfläche einer Schutzschicht erzeugt.

Der Prozeßrechner 104 arbeitet ferner mit einem Monitor 108 sowie einem Tastenfeld 110 zusammen, um Steuerbefehle entgegenzunehmen und Angaben über den Ablauf der durchgeführten Arbeiten auszugeben.

Durch den Prozeßrechner 104 wird ein erster Spindelantrieb 112 gesteuert, der auf ein zur Bearbeitung von Außenflächen besonders geeignetes Werkzeug 114 arbeitet. Ferner steuert der Prozeßrechner 104 einen Spindelantrieb 116, der ein besonders zur Bearbeitung von Innenflächen geeignetes Werkzeug 118 antreibt. Durch den Prozeßrechner 104 werden fünf Koordinatenantriebe 120, 122, 124, 126, 128 gesteuert, um das Werkzeug 114 so im Raum zu bewegen, wie dies zur Erzeugung der Außenfläche der Platzhalterschicht bzw. der Schutzschicht notwendig ist. Diese Bewegungssteuerung erfolgt unter Verwendung des im Massenspeicher 106 abgelegten berechneten digitalen Bildes dieser Flächen.

Ferner steuert der Prozeßrechner 104 fünf Koordinatenantriebe 130, 132, 134, 136 und 138, die dazu dienen, das Werkzeug 118 auf einer Fläche zu führen, die dem digitalen Bild der Außenkontur des Positivmodelles 24 entspricht, welches ebenfalls im Massenspeicher 106 abgelegt ist.

Auf diese Weise kann somit die in Figur 23 gezeigte Vorrichtung insgesamt ein formstabiles Teil herstellen, dessen Geometrie der Platzhalterschicht 26 bzw. der Schutzschicht 52 des Ausführungsbeispieles nach den Figuren 1 bis 8 entspricht.

In Variation zu der oben beschriebenen numerisch gesteuerten Herstellung eines Schutzschichtteils oder Platzhalterschichteils kann man diese auch in mechanischen Kopierschleifverfahren herstellen, wobei eine Untersetzung vom Modell zum Werkzeugstück stattfindet. Manche Halbzeuge, z.B. aus Schaum bestehende Halbzeuge lassen sich auch oft direkt manuell formen.

In Figur 24 ist schematisch die Herstellung einer kegelstumpfförmigen Schutzschichtteiles 142 ausgehend von einem blockförmigen Rohling 140 aus Keramik-Schaummaterial angedeutet, wobei dieses Material in der unteren Blockhälfte kleinere Porengröße aufweist als in der oberen Blockhälfte.

Das Schutzschichtteil 142 wird zur Herstellung eines besseren Verbundes mit zu infiltrierendem Material anschließend chemisch behandelt oder beschichtet, wobei unterschiedliche Abschnitte des Schutzschichtteiles verschieden behandelt werden können. Eine derartige Behandlung kann z.B. wiederum eine Silanisierung sein.

Über dem Schutzschichtteil 142 kann dann die Keramikzahnkrone aufgebaut werden. Dies kann so erfolgen, daß das Keramikmaterial das Schutzschichtmaterial zumindest teilweise infiltriert. Man kann dann weiter das Schutzschichtteil von der Innenseite her mit Kunststoffmaterial oder Metall infiltrieren oder ggf. siliziumorganische Materialien (infiltriert) wenigstens teilweise pyrolysieren, wie obenstehend schon beschrieben.

Das durch die Vorrichtung nach Figur 23 unter numerischer Steuerung hergestellte Teil kann aber auch als Platzhalterteil verwendet werden. In diesem Falle wird dann nach Fertigstellung der Zahnkrone 34 das Schaumstoffmaterial zur Schaffung freien Raumes für die Schutzschicht 52 entfernt. Bei Verwendung eines pyrolisierbaren Materiales kann dies durch eine Temperaturbehandlung erfolgen, bei Verwendung eines temperaturstabilen Schaummateriales durch zumindest teilweise Entfernung des Platzhalterschichtteiles. Dabei verbleibt nach vollständiger Entfernung eine Innenfläche der Zahnkrone, die gemäß den Poren des entfernten Schaumstoffmateriales sehr unregelmäßig und rauh geformt ist, ähnlich wie in Figur 8 gezeigt.

Das vollständige Entfernen der Platzhalterschicht kann z.B. chemisches Lösen oder durch Sandstrahlen der Innenfläche 36 der Zahnkrone 34 erfolgen. Wie oben beschrieben, wird dann die Innenfläche der Zahnkrone 34 vorzugsweise tribochemisch oberflächenbeschichtet oder geätzt und silanisiert, bevor dann das Schutzschichtmaterial in den zwischen Zahnkrone und Positivmodell begrenzten Formraum eingebracht wird.

In denjenigen Fällen, in denen das mit der Vorrichtung nach Figur 23 erzeugte Teil als Platzhalterschichtteil fungieren soll, das später vollständig von der Zahnkrone 34 entfernt wird, braucht die Vermessung des Positivmodelles nicht mit großer Genauigkeit vorgenommen zu werden. Es reicht dann, wenn Außen- und Innenfläche des Platzhalterteiles nur grob dem Verlauf der Präparationsfläche folgen. Unter Umständen genügt es, für die Vorgabe der Außen- und Innenflächen nur eine grobe Analyse des Positivmodelles durchzuführen, die auch von Hand oder optisch (Ausmessen eines Videobildes) erfolgen kann, wonach dann aus einem vorgegebenen Satz von Standart-Flächen die jeweils passendste ausgesucht wird.

Es versteht sich, daß auch eine spanlose formende Bearbeitung anstelle einer spanenden Bearbeitung in Betracht gezogen werden kann: Bei Schaummaterialien kann man eine Formgebung auch durch unterschiedliches lokales Zusammendrücken bewerkstelligen. Das Abtragen von Material kann auch unter Verwendung von Ultraschallerosionsgeräten oder Funkerosionsgeräten erfolgen. Schließlich können manche Schaummaterialien auch durch chemomechanische Bearbeitung geformt werden, z.B. durch lokale Säurebehandlung. All diese Arbeiten können analog zum oben beschriebenen mechanisch spanenden Bearbeiten unter numerischer Steuerung durchgeführt werden. Auch versteht sich, daß das Anpassen und Ausformen auch manuell durch spanende Bearbeitung unter Verwendung eines Technikerhandstückes erfolgen kann.

Wo die Herstellung eines selbsttragenden Platzhalterteiles oder Schutzschichtteiles aus einem sehr leichten oder instabilen Schaummaterial erfolgen soll, kann dieses zur Formgebungs-Bearbeitung vorübergehend mit einer Stabilisierungsmasse, z.B. einem Wachs oder einem wieder entfernbaren Kunststoff infiltriert werden. Diese wird dann im Falle eines Schutzschichtteiles vor Aufbau der Keramikkrone mindestens so weit aus dem geformten Schaumstoffteil entfernt, wie Keramikmasse in dieses infiltrieren soll. Das Ausmaß des Entfernung läßt sich z.B. durch die gewählte Temperaturführung vorgeben. Bei einem Platzhalterschichtteil kann die Verstärkungsmasse bis zur Entfernung der Platzhalterschicht im Schaummaterial verbleiben. Wird als Verstärkungsmaterial ein Silikon verwendet, läßt sich dieses wieder teilweise durch Pyrolyse entfernen.

Die obenstehenden Ausführungsbeispiele bezogen sich auf Zahnkronen. Es versteht sich, daß man nach dem oben geschilderten Verfahren auch größere Zahnrestaurationsteile herstellen kann, die wieder in den rißgefährdeten Bereichen mit einer Schutzschicht versehen sind. Als Beispiel wird nachstehend die Herstellung einer dreigliedrigen Brücke beschrieben.

In Figur 25 ist mit 140 ein Rohling für ein Brückenbrückengerüst-Schutzschichtteil 142 bezeichnet. Dieses dient als Füllmaterial, als Vorlage zum Aufbau des Restaurationsmateriales durch Infiltration und Überschichten sowie als Schutz gegen Rißbildung und Rißwachstum. Er ist aus einem Aluminiumoxid-Keramik-Schaummaterial hergestellt. Die Größe und Geometrie des Rohlinges 140 ist so bemessen, daß er für gängige eingliedrige Brücken gleichermaßen verwendbar ist.

Unter Verwendung eines Positivmodelles der Zahnlücke (Alveolarsattel) und der beiden benachbarten Verankerungs-Zahnstümpfe (Brückenpfeiler) wird analog wie obenstehend unter Bezugnahme auf die Figuren 1-4 beschrieben, ein Übermaß-Arbeitsmodell 144 erzeugt, welches in Figur 26 dargestellt ist. Der Rohling 140 wird manuell spanend so bearbeitet, daß man das in Figur 26 gezeigte Schutzschichtteil 142 erhält, das formschlüssig an den Zahnstümpfen 146, 148 des Arbeitsmodelles 144 anliegt und dessen Unterseite unter vorgegebenem Abstand über dem Sattel 150 des fehlenden Zahnes verläuft. Das Brückengerüst erhält so eine in Oberflachenabschnitten ggf. verkleinerte Form des späteren Brückenzwischengliedes, das durch Infiltration bzw. Überschichten mit Keramikmaterial bezüglich Form, Farbe, Transluzenz, Zahnstellung und Oberfläche zusammen mit den benachbarten Brückenankern individuell geformt, gebrannt und ausgearbeitet wird.

Alternativ kann die Einpassung des Schutzschichteiles 142 in das Arbeitsmodell 144 durch Bearbeiten unter manueller Führung des Werkzeuges 114 erfolgen.

Über dem feuerfesten Arbeitsmodell 144 und um das Schutzschichtteil 58 herum wird dann das Keramikmaterial der eingliedrigen Brücke 152 aufgebaut, wie in Figur 27 dargestellt. Dabei verbleibt zwischen der Unterseite des Keramikmateriales und der Oberseite des Arbeitsmodelles 144 im Übergangsbereich zwischen dem mittleren und rechten dem prämolaren Brückenpfeiler zugeordneten Brückensegment ein Zwischenraum 154 zur Oberseite des Arbeitsmodelles 144, wie aus Figur 27 und 28 näher ersichtlich.

Nach Brennen des Keramikmateriales wird das Brückenteil 152 auf das Positivmodell 156 der Restaurationsregion aufgeschoben, welches sich vom Arbeitsmodell 144 auf den Zahnstümpfen 158, 160 um die Platzhalterschicht unterscheidet. Man erhält so zwischen dem linken Brückensegment und dem Zahnstumpf 158 und dem rechten Brückensegment und dem Zahnstumpf 160 wieder Formräume, die ähnlich mit Kunststoffmaterial gefüllt werden, wie obenstehend unter Bezugnahme auf Figur 5 im einzelnen beschrieben, wodurch man Schutzschichten 162, 164 erhält. Ein Teil dieses Kunststoffmateriales tritt in den in Figur 28 näher gezeigten Zwischenraum 154 ein und sorgt so im Übergangsbereich zwischen dem mittleren und rechten Brückensegment für einen erhöhten Schutz gegen Rißbildung durch eine zusätzliche, hier dicker gezeigte Schutzschicht 166. Der entsprechende Abschnitt der fertigen Brücken 152 ist in Figur 30 vergrößert wiedergegeben.

Im Übergangsbereich zwischen dem linken und dem mittleren Brückensegment läuft dagegen das Keramikmaterial bis zur Unterseite der Brücke.

Das Sattelsegment des Positivmodelles 156 ist keine Gießform oder sonstige direkt auf ein zu formendes Material einwirkende Form sondern eine Lehre, anhand welcher der Zahntechniker die Formgestaltung der Unterseite der Brücke vornimmt. Hierbei hält er die aus der Zeichnung ersichtlichen Abstände zwischen der Brückenunterseite und der Oberseite des Sattelsegmentes ein.

Figur 32 zeigt die eingegliederte Brücke auf den Zahnstümpfen 170, 172. Die Befestigung der Brücke an den Zahnstümpfen erfolgt über Fügematerialschichten 174, 176, z.B. Zementschichten.

Bei der oben beschriebenen Brücke sind Spannungsbrüche in dem großen Keramikvolumen des mittleren Brückensegmentes (durch das Schutzschichtteil 142) und in der Nachbarschaft der Befestigungsflächen an den Zahnstümpfen 170, 172 durch die Schutzschichten 162, 164 vermieden. Dabei wurde eine besondere Verstärkung im besonders belasteten Übergangsbereich zwischen mittlerem und rechtem, dem prämolaren Brückenpfeiler zugeordnetem Brückensegment vorgesehen (Schutzschicht 166).

Bei beidseitig besonders belasteten Brücken kann man auch im Übergangsbereich zwischen linkem und mittlerem Brückensegment eine solche zusätzliche Schutzschicht vorsehen, welche der Schutzschicht 166 entspricht.

Umgekehrt kann man bei weniger belasteten Brücken eine zusätzliche Schutzschicht 166 im Übergangsbereich zwischen dem mittleren Brückensegment und den randständigen Brückensegmenten weglassen.

Bei den oben beschriebenen keramischen Zahnrestaurationsteilen war die Schutzschicht entweder im Inneren des Keramikvolumens vorgesehen (mittleres Brückensegment der Brücke nach Figur 29) oder an becherförmigen Innenflächen von Kronenteilen oder kronenähnlichen Brückensegmenten vorgesehen. Bei anderen Zahnrestaurationsteilen, wo die besonders rißanfälligen Materialbereiche anders verlaufen, verlaufen auch die Schutzschichten entsprechend anders. Als ein Beispiel für ein solches anderes Zahnrestaurationsteil ist in Figur 33 ein aus Dentalkeramik hergestelltes Inlay gezeigt (ähnliches Vorgehen bei Onlay, Overlay, Teilkrone). Dieses ist in eine von der Kaufläche ausgehende Ausnehmung (Kavität) 178 eines Molars unter Verwendung einer Zementschicht 180 fest eingesetzt. In der mod-Längsfissur ist eine etwa keilförmige Vertiefung 182 vorgesehen, welche mit einem Schutzschichtmaterial ausgefüllt ist. Die Schutzschicht 184 läuft somit in die Kaufläche frei aus und ist dort entsprechend dem gewünschten Fissurenrelief gestaltet.

Vorzugsweise sind wenigstens Teile der mod-Längsfissur des Inlays, Overlays oder der Teilkrone mit der Schutzschicht versehen.

Bei Kronen können Schutzschichten z.B. außen (zusätzlich) entweder in Bereichen der Fissuren (Seitenzähne und Frontzähne) oder außen (z.B. im palatinalen Bereich von Frontzähnen vorgesehen werden.

Die oben beschriebenen Beispiele für mit Schutzschichten versehene Zahnrestaurationsteile waren zur Eingliederung an natürlichen Zahnstümpfen bestimmt. Es versteht sich, daß derartige Schutzschichten gleichermaßen für Zahnrestaurationsteile nützlich sind, welche auf künstlichen Zahnstümpfen befestigt werden, z.B. implantierten Zahnstümpfen. Da Implantat-Zahnstümpfe in der Regel konfektionierte Anschlußflächen zur Verbindung z.B. mit Stumpfaufbauten oder anderen Suprakonstruktionen aufweisen, kann man Platzhalterschichtteile oder Schutzschichtteile als standardisierte Teile bereitstellen; eine spezielle Formgebung zu mindest der Innenfläche dieser Teile ist dann nicht notwendig, die Außenfläche ist ggf. gemäß den individuell angetroffenen Verhältnissen zu bearbeiten. Die Erfindung wurde oben stehend bezugnehmend auf Zahnrestaurationsteile erläutert. Es versteht sich, daß man nach dem geschilderten Prinzip auch andere medizinische Keramikimplantate oder Prothesen realisieren kann. Diese zeichnen sich dadurch aus, daß sie leichter sind als konventionelle Implantate und kompaktere Geometrie haben können. Anwendbar ist die Erfindung insbesondere auch auf Skelettimplantate.

Im Falle von Implantaten können Schutzschichten auch vor dem Hintergrund einer elastischen Verformung z.B. zum Inaktivieren von problematischen Drehmomenten jeweils bezogen auf die Implantationslängsachse als Dämpfungselement ausgebildet sein. So kann z.B. häufig ein Innensechskant oder Außensechskant des Implantates mit einer komplementären Schutzschicht eines Restaurationsteiles so verbunden sein, daß diese zugleich die Dämpfungsfunktion erfüllt.

Figur 34 zeigt schematisch einen transversalen Schnitt durch die Verbindungsstelle zwischen einem schematisch angedeuteten Implantat 186 und einem mit diesem verbundenen Prothesenteil 188.

Am Ende des Implantats ist ein Innensechskant 190 vorgesehen, während das Prothesenteil 188 einen Außensechskant 192 aufweist. Über dem Außensechskant 192 befindet sich eine Schutzschicht 194, welche aufgrund ihrer Elastizität geringfügige Verdrehungen zwischen dem Innensechskant 190 und dem Außensechskant 192 zuläßt, wobei durch die innere Dämpfung der Schutzschicht 194 derartige Relativbewegungen stark gedämpft werden.

## Patentansprüche

1. Zahnrestaurationsteil aus Keramikmaterial (58) mit mindestens einer Außenfläche und mit mindestens einer mit einer präparierten Fläche (12) eines natürlichen oder implantierten Zahnteiles (10) verbindbaren Befestigungsfläche (36), dadurch gekennzeichnet, daß die zug- und/ oder spannungsbeanspruchten Oberflächen und/oder Oberflächenrandzonen zumindest teilweise mit einer eine Rißbildung und/oder ein Wachstum von Rissen im Keramikmaterial (58) entgegenwirkenden Schutzschicht (52) verbunden sind.

2. Zahnrestaurationsteil nach Anspruch 1, dadurch gekennzeichnet, daß die Schutzschicht (52) elastisch oder plastisch verformbar ist.

3. Zahnrestaurationsteil nach Anspruch 1 oder 2, dadurch gekennzeichnet, daß die Schutzschicht (52) hydrolysestabil ist.

4. Zahnrestaurationsteil nach einem der Ansprüche 1 bis 3, dadurch gekennzeichnet, daß es eine Einzelzahnkrone (34), die allein steht oder im Kronenverbund steht oder als Doppelkrone oder Brückenanker steht und seine Innenfläche (36) wenigstens teilweise mit einer Schutzschicht (52) versehen ist.

5. Zahnrestaurationsteil nach einem der Ansprüche 1 bis 4, dadurch gekennzeichnet, daß die Schutzschicht (52) und das Keramikmaterial (58) an ihrer Berührfläche (36, 56) über komplementäre Oberflächenrauhigkeiten miteinander verhakt sind.

6. Zahnrestaurationsteil nach einem der Ansprüche 1 bis 5, dadurch gekennzeichnet, daß die Schutzschicht (52) metallisches oder keramisches Schaummaterial umfaßt.

7. Zahnrestaurationsteil nach Anspruch 6, dadurch gekennzeichnet, daß die Schutzschicht (52) aus einem Aluminiumoxid-Schaummaterial besteht.

8. Zahnrestaurationsteil nach Anspruch 7, dadurch gekennzeichnet, daß das Aluminiumoxid-Schaummaterial ein dicht gesintertes Aluminiumoxid-Material ist.

9. Zahnrestaurationsteil nach einem der Ansprüche 5 bis 8, dadurch gekennzeichnet, daß die Anzahl der Poren (63, 64) pro Zentimeter zwischen 12 und 48, vorzugsweise zwischen 20 und 35, nochmals bevorzugt zwischen 25 und 32 beträgt.

10. Zahnrestaurationsteil nach einem der Ansprüche 5 bis 9, dadurch gekennzeichnet, daß die Größe der Poren (63, 64) in zur Innenfläche (36) des Zahnrestaurationsteiles (34) senkrechter Richtung zu- oder abnimmt.

11. Zahnrestaurationsteil nach einem der Ansprüche 5 bis 10, dadurch gekennzeichnet, daß das Zahnrestaurationsteil (34) bildende Keramikmaterial (58) zumindest einen Teil der Poren (63, 64) des Schaummateriales ausfüllt.

12. Zahnrestaurationsteil nach einem der Ansprüche 5 bis 11, dadurch gekennzeichnet, daß das Schutzschichtmaterial zumindest einen Teil der Poren (63, 64) des Schaummateriales ausfüllt.

13. Zahnrestaurationsteil nach einem der Ansprüche 1 bis 10, dadurch gekennzeichnet, daß die Schutzschicht (52) zumindest teilweise aus wenigstens einem Kunststoff und/ oder einem natürlichen und/oder künstlichen Harz besteht.

14. Zahnrestaurationsteil nach Anspruch 13, dadurch gekennzeichnet, daß der Kunststoff ein thermoplastischer oder duroplastischer Kunststoff ist.

15. Zahnrestaurationsteil nach Anspruch 14, dadurch gekennzeichnet, daß der Kunststoff ausgewählt ist aus den Gruppen der Polysulfone, der Acrylate oder aus siliziumorganischen Polymeren.

16. Zahnrestaurationsteil nach einem der Ansprüche 13 bis 15, dadurch gekennzeichnet, daß der Kunststoff ein Gemenge verschiedener Kunststoffe ist.

17. Zahnrestaurationsteil nach einem der Ansprüche 1 bis 16, dadurch gekennzeichnet, daß die Schutzschicht (52) wenigstens teilweise aus einem durch Partikel und/oder Fasern verstärkten Kunststoff und/oder Harz besteht.

18. Zahnrestaurationsteil nach einem der Ansprüche 1 bis 17, dadurch gekennzeichnet, daß die Dicke der Schutzschicht (52) zwischen 0,1 mm und 1,0 mm, vorzugsweise zwischen 0,1 mm und 0,6 mm, nochmals vorzugsweise zwischen 0,3 mm und 0,6 mm beträgt.

19. Verfahren zum Herstellen eines keramischen Zahnrestaurationsteiles gemäß einem der Ansprüche 1 bis 18, gekennzeichnet durch folgende Verfahrensschritte:
a) Herstellen eines Positivmodelles der präparierten Fläche;
b) Einbringen einer Platzhalterschicht auf denjenigen Abschnitten des Positivmodelles, die zug- oder scherspannungsbeanspruchten Oberflächen und/oder Oberflächenrandzonen des Zahnrestaurationsteiles entsprechen;
c) Formen des Keramikmateriales über dem mit der Platzhalterschicht versehenen Positivmodell und Brennen des Keramikmateriales, ggf. in mehreren übereinanderliegenden Teilschichten;
d) Entfernen der Platzhalterschicht;
e) Aufsetzen des gebrannten Keramikformteiles auf das Positivmodell;
f) Einbringen eines plastisch verformbaren und erhärtenden oder härtbaren Schutzschichtmateriales in den Formraum, welcher zwischen dem gebrannten Keramikformteil und dem Positivmodell begrenzt ist;
g) Härten des Schutzschichtmateriales.

20. Verfahren nach Anspruch 19, dadurch gekennzeichnet, daß die Platzhalterschicht aus einem bei Brennbedingungen stabilen Material, insbesondere einer Platinlegierung besteht.

21. Verfahren nach Anspruch 20, dadurch gekennzeichnet, daß die Platzhalterschicht aus einem metallischen oder keramischen Schaummaterial besteht und nach Brennen des Keramikformteiles und vor Aufbringung der Schutzschicht teilweise oder vollständig entfernt wird.

22. Verfahren nach Anspruch 21, dadurch gekennzeichnet, daß die Platzhalterschicht aus einem metallischen oder keramischen Schaummaterial besteht und auf dem Keramikformteil verbleibt.

23. Verfahren nach Anspruch 21 oder 22, dadurch gekennzeichnet, daß die Platzhalterschicht bzw. ein nach ihrem Entfernen in der Oberfläche des Keramikformteiles verbleibendes Relief wenigstens teilweise mit dem Schutzschichtmaterial infiltriert wird.

24. Verfahren nach einem der Ansprüche 21 bis 23, dadurch gekennzeichnet, daß die Platzhalterschicht beim Aufbau des Keramikmateriales über der Platzhalterschicht zumindest teilweise mit dem Keramikmaterial infiltriert wird.

25. Verfahren zum Herstellen eines keramischen Zahnrestaurationsteiles nach einem der Ansprüche 1 bis 18, gekennzeichnet durch folgende Verfahrensschritte:
a) Herstellen eines Positivmodelles der präparierten Fläche;
b) Anbringen einer Platzhalterschicht auf denjenigen Abschnitten des Positivmodelles, die zug- oder scherspannungsbeanspruchten Oberflächen und/oder Oberflächenrandzonen des Restaurationsteiles entsprechen;
c) Herstellung einer Doublette des mit der Platzhalterschicht versehenen Positivmodelles;
d) Formen des Keramikmateriales über der Doublette des Positivmodelles und Brennen des Keramikmateriales, ggf. in mehreren übereinanderliegenden Teilschichten;
e) Entfernen der Platzhalterschicht vom Positivmodell;
f) Aufsetzen des Keramikformteiles auf das Positivmodell;
g) Einbringen eines plastisch verformbaren und erhärtenden oder härtbaren Schutzschichtmateriales in den Formraum, der zwischen dem Keramikformteil und dem Positivmodell begrenzt ist;
h) Aushärten des Schutzschichtmateriales.

26. Verfahren nach Anspruch 25, dadurch gekennzeichnet,
daß die Platzhalterschicht aus einem schmelzbaren oder löslichen Material besteht und das Entfernen der Platzhalterschicht durch Einwirkung von Temperatur oder eines Lösungsmittels erfolgt.

27. Verfahren zum Herstellen eines keramischen Zahnrestaurationsteiles nach einem der Ansprüche 1 bis 18, gekennzeichnet durch folgende Verfahrensschritte:
a) Herstellen eines Positivmodelles der Zahnfläche;
b) Herstellen einer Aufmaß-Doublette des Positivmodelles;
c) Formen des Keramikmateriales über der Doublette des Positivmodelles;
d) Brennen des Keramikmateriales;
e) Aufsetzen des Keramikformteiles auf das Positivmodell;
f) Einbringen eines plastisch verformbaren und erhärtenden oder härtbaren Schutzschichtmateriales in den von dem Keramikformteil und dem Positivmodell begrenzten Raum;
g) Aushärten des Schutzschichtmateriales.

28. Verfahren nach Anspruch 26, dadurch gekennzeichnet, daß zur Herstellung der Aufmaß-Doublette ein Negativabdruck des Positivmodelles mit einer Modelliermasse verwendet wird, die bei ihrer Verfestigung eine Volumensverminderung erfährt, und die Aufmaß-Doublette durch Ausgießen der Negativform erfolgt.

29. Verfahren zum Herstellen eines keramischen Zahnrestaurationsteiles nach einem der Ansprüche 1 bis 18, gekennzeichnet durch folgende Verfahrensschritte:
a) Herstellen eines Positivmodelles der Zahnfläche;
b) Formen des Keramikmateriales über dem Positivmodell;
c) Brennen des Keramikmateriales;
d) Entfernen von Keramikmaterial auf denjenigen Abschnitten des Keramikformteiles, die zug- oder scherspannungsbeanspruchten Oberflächen und/oder Oberflächenrandzonen des keramischen Zahnrestaurationsteiles entsprechen;
e) Aufsetzen des Keramikformteiles auf das Positivmodell;
f) Einbringen eines plastisch verformbaren und erhärtenden oder härtbaren Schutzschichtmateriales in den zwischen dem Keramikformteil und dem Positivmodell begrenzten Raum;
g) Aushärten des Schutzschichtmateriales.

30. Verfahren zum Herstellen eines keramischen Zahnrestaurationsteiles nach einem der Ansprüche 1 bis 18, gekennzeichnet durch folgende Verfahrensschritte:
a) Herstellen eines Positivmodelles der präparierten Fläche;
b) Vermessen der präparierten Fläche auf dem Positivabdruck und Abspeichern der gemessenen Flächenkontur;
c) Formen eines Schutzschichtteiles oder Platzhalterteiles unter numerischer Steuerung, wobei dessen eine Begrenzungsfläche der abgespeicherten Flächenkontur entspricht und dessen zweite Begrenzungsfläche einer zweiten, beabstandeten Flächenkontur entspricht, die aus der ersten Flächenkontur abgeleitet ist, z. B. durch Parallelverschiebung;
d) Formen des Keramikmateriales über der geformten Schutzschicht;
e) Brennen des Keramikmateriales.

31. Verfahren zum Herstellen eines keramischen Zahnrestaurationsteiles nach einem der Ansprüche 1 bis 18, gekennzeichnet durch folgende Verfahrensschritte:
a) Herstellen eines Positivmodelles der präparierten Fläche;
b) Ausmessen des Positivmodelles und Abspeichern seiner Flächenkontur;
c) Formen von Keramikmaterial über einem Schutzschichtmaterialteil, welches vorzugsweise aus metallischem oder keramischen Schaummaterial besteht;
und
d) Brennen des Keramikmateriales;
e) Ausarbeiten einer Innenfläche in dem Schutzschichtmaterialteil unter numerischer Steuerung gemäß der abgespeicherten Flächenkontur;
oder
d') Ausarbeiten einer Innenfläche in dem Schutzschichtmaterial unter numerischer Steuerung gemäß der abgespeicherten Flächenkontur;
e') Brennen des Keramikmateriales.

32. Verfahren nach Anspruch 30 oder 31, dadurch gekennzeichnet, daß die Poren des aus Schaummaterial bestehenden Schutzschichtmaterialteiles zumindest teilweise mit einem Kunststoffmaterial infiltriert werden.

33. Verfahren nach einem der Ansprüche 19 bis 32, dadurch gekennzeichnet, daß die mit dem Schutzschichtmaterial zu beschichtenden Flächen des Keramikformteiles zumindest teilweise durch tribochemische Oberflächenbehandlung mit Silikat angereichert oder angeätzt und/oder silanisiert werden.

34. Verfahren nach einem der Ansprüche 19 bis 33, dadurch gekennzeichnet, daß das Positivmodell vor dem Aufsetzen des Keramikformteiles und dem Formen des Schutzschichtmateriales mit einem Trennmittel versehen wird.

35. Verfahren nach einem der Ansprüche 19 bis 34, dadurch gekennzeichnet, daß das Formen des Schutzschichtmateriales in dem durch das Keramikformteil und den Positivabdruck begrenzten Raum unter gleichzeitiger Einwirkung von Wärme und/oder mechanischer Schwingung, z. B. Ultraschall, und/oder Druck erfolgt.

36. Verfahren nach einem der Ansprüche 19 bis 35, dadurch gekennzeichnet, daß das Schutzschichtmaterial zumindest teilweise aus siliziumorganischen Polymeren besteht und zumindest teilweise keramisiert wird.

37. Medizinische Prothese, dadurch gekennzeichnet, daß sie ein Schaummaterialvolumen (52; 142) umfaßt, welches zumindest teilweise mit einem Infiltrationsmaterial (60, 61; 58; 166) imprägniert ist.

38. Prothese nach Anspruch 37, dadurch gekennzeichnet, daß das Schaummaterialvolumen (52; 142) aus keramischem oder metallischem Schaummaterial besteht.

39. Prothese nach Anspruch 37 oder 38, dadurch gekennzeichnet, daß das Infiltrationsmaterial ein Kunststoffmaterial (60; 166) oder ein keramisches Material (61; 58) ist.

40. Prothese nach einem der Ansprüche 37 bis 39, dadurch gekennzeichnet, daß das Infiltrationsmaterial elastisch oder plastisch verformbar ist.

## Claims

1. Tooth restoration part of ceramic material (58) with at least one outer surface and with at least one fixing surface (36) which can be joined to a prepared surface (12) of a natural or implanted tooth part (10), characterized in that the surfaces and/or surface edge zones subject to tensile and/or [shear] stress are at least partially joined to a protective layer (52) counteracting crack formation and/or propagation of cracks in the ceramic material (58).

2. Tooth restoration part according to Claim 1, characterized in that the protective layer (52) is elastically or plastically deformable.

3. Tooth restoration part according to Claim 1 or 2, characterized in that the protective layer (52) is stable to hydrolysis.

4. Tooth restoration part according to one of Claims 1 to 3, characterized in that it [is] a single crown (34) which stands alone or is part of a compound crown or stands as double crown or bridge anchor and its inner surface (36) is provided at least partially with a protective layer (52).

5. Tooth restoration part according to one of Claims 1 to 4, characterized in that the protective layer (52) and the ceramic material (58) are hooked together at their contact surface (36, 56) by means of complementary surface roughnesses.

6. Tooth restoration part according to one of Claims 1 to 5, characterized in that the protective layer (52) comprises metallic or ceramic foam material.

7. Tooth restoration part according to Claim 6, characterized in that the protective layer (52) consists of an aluminium oxide foam material.

8. Tooth restoration part according to Claim 7, characterized in that the aluminium oxide foam material is a densely sintered aluminium oxide material.

9. Tooth restoration part according to one of Claims 5 to 8, characterized in that the number of pores (63, 64) per centimetre is between 12 and 48, preferably between 20 and 35, even more preferably between 25 and 32.

10. Tooth restoration part according to one of Claims 5 to 9, characterized in that the size of the pores (63, 64) increases or decreases in the direction perpendicular to the inner surface (36) of the tooth restoration part (34).

11. Tooth restoration part according to one of Claims 5 to 10, characterized in that the ceramic material (58) forming the tooth restoration part (34) fills at least a part of the pores (63, 64) of the foam material.

12. Tooth restoration part according to one of Claims 5 to 11, characterized in that the protective layer material fills at least a part of the pores (63, 64) of the foam material.

13. Tooth restoration part according to one of Claims 1 to 10, characterized in that the protective layer (52) consists at least partially of at least one plastic and/or one natural and/or synthetic resin.

14. Tooth restoration part according to Claim 13, characterized in that the plastic is a thermoplastic or thermoset plastic.

15. Tooth restoration part according to Claim 14, characterized in that the plastic is selected from the group of the polysulfones, acrylates or from organosilicon polymers.

16. Tooth restoration part according to one of Claims 13 to 15, characterized in that the plastic is a mixture of different plastics.

17. Tooth restoration part according to one of Claims 1 to 16, characterized in that the protective layer (52) consists at least partially of a plastic and/or resin reinforced with particles and/or fibres.

18. Tooth restoration part according to one of Claims 1 to 17, characterized in that the thickness of the protective layer (52) is between 0.1 mm and 1.0 mm, preferably between 0.1 mm and 0.6 mm, even more preferably between 0.3 mm and 0.6 mm.

19. Process for producing a ceramic tooth restoration part according to one of Claims 1 to 18, characterized by the following process steps:
a) production of a positive model of the prepared surface;
b) application of a holding layer on those sections of the positive model which correspond to surfaces and/or surface edge zones of the tooth restoration part which are subject to tensile or shear stress;
c) moulding of the ceramic material over the positive model provided with the holding layer and firing of the ceramic material, optionally in several superimposed individual layers;
d) removal of the holding layer;
e) fitting of the fired ceramic moulded part onto the positive model;
f) insertion of a plastically deformable and setting or settable protective layer material into the moulding space which is delimited between the fired ceramic moulded part and the positive model;
g) setting of the protective layer material.

20. Process according to Claim 19, characterized in that the holding layer consists of a material which is stable under firing conditions, particularly a platinum alloy.

21. Process according to Claim 20, characterized in that the holding layer consists of a metallic or ceramic foam material and is partially or completely removed after firing the ceramic moulded part and before applying the protective layer.

22. Process according to Claim 21, characterized in that the holding layer consists of a metallic or ceramic foam material and remains on the ceramic moulded part.

23. Process according to Claim 21 or 22, characterized in that the holding layer and/or a relief remaining in the surface of the ceramic moulded part after its removal is at least partially infiltrated with the protective layer material.

24. Process according to one of Claims 21 to 23, characterized in that the holding layer is at least partially infiltrated with the ceramic material when the ceramic material is built up over the holding layer.

25. Process for producing a ceramic tooth restoration part according to one of Claims 1 to 18, characterized by the following process steps:
a) production of a positive model of the prepared surface;
b) application of a holding layer on those sections of the positive model which correspond to surfaces and/or surface edge zones of the tooth restoration part which are subject to tensile or shear stress;
c) production of a duplicate of the positive model provided with the holding layer;
d) moulding of the ceramic material over the duplicate of the positive model and firing of the ceramic material, optionally in several superimposed individual layers;
e) removal of the holding layer from the positive model;
f) fitting of the ceramic moulded part onto the positive model;
g) insertion of a plastically deformable and setting or settable protective layer material into the moulding space which is delimited between the ceramic moulded part and the positive model;
h) setting of the protective layer material.

26. Process according to Claim 25, characterized in that the holding layer consists of a fusible or soluble material and the holding layer is removed by the effect of temperature or a solvent.

27. Process for producing a ceramic tooth restoration part according to one of Claims 1 to 18, characterized by the following process steps:
a) production of a positive model of the tooth surface;
b) production of an oversize duplicate of the positive model;
c) moulding of the ceramic material over the duplicate of the positive model;
d) firing of the ceramic material;
e) fitting of the ceramic moulded part onto the positive model;
f) insertion of a plastically deformable and setting or settable protective layer material into the space delimited by the ceramic moulded part and the positive model;
g) setting of the protective layer material.

28. Process according to Claim 26, characterized in that to produce the oversize duplicate, a negative impression of the positive model is used with a modelling compound which undergoes a volume reduction when it solidifies and the oversize duplicate is produced by filling of the negative mould.

29. Process for producing a ceramic tooth restoration part according to one of Claims 1 to 18, characterized by the following process steps:
a) production of a positive model of the tooth surface;
b) moulding of the ceramic material over the positive model;
c) firing of the ceramic material;
d) removal of ceramic material on those sections of the ceramic moulded part which correspond to surfaces and/or surface edge zones of the ceramic tooth restoration part which are subject to tensile and/or shear stress;
e) fitting of the ceramic moulded part onto the positive model;
f) insertion of a plastically deformable and setting or settable protective layer material into the space delimited between the ceramic moulded part and the positive model;
g) setting of the protective layer material.

30. Process for producing a ceramic tooth restoration part according to one of Claims 1 to 18, characterized by the following process steps:
a) production of a positive model of the prepared surface;
b) measurement of the prepared surface on the positive impression and storage of the surface contour measured;
c) moulding of a protective layer part or holding part using numerical control, wherein its one boundary surface corresponds to the stored surface contour and its second boundary surface corresponds to a second surface contour a distance away, which is derived from the first surface contour, by parallel displacement for example;
d) moulding of the ceramic material over the moulded protective layer;
e) firing of the ceramic material.

31. Process for producing a ceramic tooth restoration part according to one of Claims 1 to 18, characterized by the following process steps:
a) production of a positive model of the prepared surface;
b) measurement of the positive model and storage of its surface contour;
c) moulding of ceramic material over a protective layer material part which preferably consists of metallic or ceramic foam material;
and
d) firing of the ceramic material;
e) finishing of an inner surface in the protective layer material part using numerical control according to the stored surface contour;
or
d') finishing of an inner surface in the protective layer material using numerical control according to the stored surface contour;
e') firing of the ceramic material.

32. Process according to Claim 30 or 31, characterized in that the pores of the protective layer material part consisting of foam material are at least partially infiltrated with a plastics material.

33. Process according to one of Claims 19 to 32, characterized in that the surfaces of the ceramic moulded part to be coated with the protective layer material are at least partially enriched with silicate by tribochemical surface treatment or etched and/or silanized.

34. Process according to one of Claims 19 to 33, characterized in that the positive model is provided with a release agent prior to the fitting of the ceramic moulded part and the moulding of the protective layer material.

35. Process according to one of Claims 19 to 34, characterized in that the moulding of the protective layer material in the space delimited by the ceramic moulded part and the positive impression takes place under the simultaneous effect of heat and/or mechanical oscillation, e.g. ultrasound, and/or pressure.

36. Process according to one of Claims 19 to 35, characterized in that the protective layer material consists at least partially of organosilicon polymers and is at least partially ceramized.

37. Medical prosthesis, characterized in that it comprises a foam material volume (52; 142) which is at least partially impregnated with an infiltration material (60, 61; 58; 166).

38. Prosthesis according to Claim 37, characterized in that the foam material volume (52; 142) consists of ceramic or metallic foam material.

39. Prosthesis according to Claim 37 or 38, characterized in that the infiltration material is a plastics material (60; 166) or a ceramic material (61; 58).

40. Prosthesis according to one of Claims 37 to 39, characterized in that the infiltration material is elastically or plastically deformable.

## Revendications

1. Elément de restauration dentaire en matériau céramique (58) avec au moins une surface extérieure et avec au moins une surface de fixation (36) pouvant être reliée à une surface préparée (12) d'un élément de dent naturel ou implanté (10), caractérisé en ce que les surfaces et/ou les zones de bord des surfaces subissant des contraintes et/ou des tractions sont reliées au moins pour partie à une couche de protection (52) s'opposant à une formation de fissures et/ou à un développement de fissures dans le matériau céramique (58).

2. Elément de restauration dentaire selon la revendication 1, caractérisé en ce que la couche de protection (52) peut être déformée élastiquement ou plastiquement.

3. Elément de restauration dentaire selon la revendication 1 ou 2, caractérisé en ce que la couche de protection (52) est stable à l'hydrolyse.

4. Elément de restauration dentaire selon l'une des revendications 1 à 3, caractérisé en ce qu'il est prévu une couronne dentaire unitaire (34) qui se trouve seule ou dans un ensemble à couronnes ou fait office de couronne double ou d'ancrage de bridge et sa surface intérieure (36) est au moins partiellement dotée d'une couche de protection (52).

5. Elément de restauration dentaire selon l'une des revendications 1 à 4, caractérisé en ce que la couche de protection (52) et le matériau céramique (58) sont accrochés entre eux au niveau de leur surface de contact (36, 56) par l'intermédiaire d'irrégularités de surface complémentaires.

6. Elément de restauration dentaire selon l'une des revendications 1 à 5, caractérisé en ce que la couche de protection (52) comprend un matériau mousse métallique ou céramique.

7. Elément de restauration dentaire selon la revendication 6, caractérisé en ce que la couche de protection (52) se compose d'un matériau mousse d'oxyde d'aluminium.

8. Elément de restauration dentaire selon la revendication 7, caractérisé en ce que le matériau mousse d'oxyde d'aluminium est un matériau d'oxyde d'aluminium fritté.

9. Elément de restauration dentaire selon l'une des revendications 5 à 8, caractérisé en ce que la quantité de pores (63, 64) par centimètre est comprise entre 12 et 48, de préférence entre 20 et 35, de façon encore plus préférentielle, entre 25 et 32.

10. Elément de restauration dentaire selon l'une des revendications 5 à 9, caractérisé en ce que la grandeur des pores (63, 64) augmente ou diminue dans la direction perpendiculaire à la surface intérieure (36) de l'élément de restauration dentaire (34).

11. Elément de restauration dentaire selon l'une des revendications 5 à 10, caractérisé en ce que le matériau céramique (58) composant l'élément de restauration dentaire (34) remplit au moins une partie des pores (63, 64) du matériau mousse.

12. Elément de restauration dentaire selon l'une des revendications 5 à 11, caractérisé en ce que le matériau de la couche de protection remplit au moins une partie des pores (63, 64) du matériau mousse.

13. Elément de restauration dentaire selon l'une des revendications 1 à 10, caractérisé en ce que la couche de protection (52) se compose au moins pour partie d'un matériau synthétique et/ou d'une résine naturelle et/ou d'une résine synthétique.

14. Elément de restauration dentaire selon la revendication 13, caractérisé en ce que le matériau synthétique est un matériau synthétique thermoplastique ou thermodurcissable.

15. Elément de restauration dentaire selon la revendication 14, caractérisé en ce que le matériau synthétique est sélectionné parmi les groupes des polysulfones, des acrylates ou à partir de polymères de silicium organiques.

16. Elément de restauration dentaire selon l'une des revendications 13 à 15, caractérisé en ce que le matériau synthétique est un mélange de divers matériaux synthétiques.

17. Elément de restauration dentaire selon l'une des revendications 1 à 16, caractérisé en ce que la couche de protection (52) se compose au moins pour partie d'un matériau synthétique et/ou d'une résine renforcé(e) par des fibres et/ou des particules

18. Elément de restauration dentaire selon l'une des revendications 1 à 17, caractérisé en ce que l'épaisseur de la couche de protection (52) est comprise entre 0,1 mm et 1,00 mm, de préférence entre 0,1 mm et 0,6 mm, et de façon encore plus préférentielle, entre 0,3 mm et 0,6 mm.

19. Procédé de fabrication d'un élément de restauration dentaire céramique selon l'une des revendications 1 à 18, caractérisé en ce qu'il comprend les étapes opératoires suivantes, consistant :
a) à fabriquer un modèle positif de la surface préparée ;
b) à déposer une couche de maintien à distance sur les segments du modèle positif qui correspondent aux surfaces subissant des contraintes de traction et/ou de cisaillement et/ou aux zones des bords des surfaces de l'élément de restauration ;
c) à façonner le matériau céramique sur le modèle positif doté de la couche de maintien à distance et à cuire le matériau céramique, éventuellement en plusieurs couches partielles disposées les unes sur les autres ;
d) à retirer la couche de maintien à distance ;
e) à mettre en place l'élément de moulage céramique cuit sur le modèle positif ;
f) à introduire un matériau de couche de protection plastiquement déformable et durcissant ou durcissable dans l'espace de moulage, lequel est limité entre l'élément de moulage céramique cuit et le modèle positif ;
g) à faire durcir le matériau de la couche de protection.

20. Procédé selon la revendication 19, caractérisé en ce que la couche de maintien à distance se compose d'un matériau stable dans des conditions de cuisson, en particulier d'un alliage platiné.

21. Procédé selon la revendication 20, caractérisé en ce que la couche de maintien à distance se compose d'un matériau mousse métallique ou céramique et en ce qu'elle est enlevée pour tout ou partie, après cuisson de l'élément de moulage céramique et avant application de la couche de protection.

22. Procédé selon la revendication 21, caractérisé en ce que la couche de maintien à distance se compose d'un matériau mousse métallique ou céramique et en ce qu'elle demeure sur l'élément de moulage céramique.

23. Procédé selon la revendication 21 ou 22, caractérisé en ce que la couche de maintien à distance ou un relief demeurant après son enlèvement en surface de l'élément de moulage céramique est infiltré(e) au moins pour partie avec le matériau de la couche de protection.

24. Procédé selon l'une des revendications 21 à 23, caractérisé en ce que la couche de maintien à distance lors de la constitution du matériau céramique sur la couche de maintien à distance est infiltrée au moins pour partie avec le matériau céramique.

25. Procédé de fabrication d'un élément de restauration dentaire céramique selon l'une des revendications 1 à 18, caractérisé en ce qu'il comprend les étapes opératoires suivantes, consistant :
a) à fabriquer un modèle positif de la surface préparée ;
b) à déposer une couche de maintien à distance sur les segments du modèle positif qui correspondent aux surfaces subissant des contraintes de traction ou de cisaillement et/ou aux zones des bords des surfaces de l'élément de restauration ;
c) à construire un double du modèle positif doté de la couche de maintien à distance ;
d) à façonner le matériau céramique sur le double du modèle positif et à cuire le matériau céramique, éventuellement en plusieurs couches partielles disposées les unes sur les autres ;
e) à retirer du modèle positif la couche de maintien à distance ;
f) à mettre en place l'élément de moulage céramique sur le modèle positif ;
g) à introduire un matériau de couche de protection plastiquement déformable et durcissant ou durcissable dans l'espace de moulage, qui est limité entre l'élément de moulage céramique et le modèle positif ;
h) à faire durcir le matériau de la couche de protection.

26. Procédé selon la revendication 25, caractérisé en ce que la couche de maintien à distance se compose d'un matériau fusible ou décapable et en ce que l'enlèvement de la couche de maintien à distance est réalisé par l'action de la température ou d'un agent décapant.

27. Procédé de fabrication d'un élément de restauration dentaire céramique selon l'une des revendications 1 à 18, caractérisé en ce qu'il comprend les étapes opératoires suivantes, consistant :
a) à fabriquer un modèle positif de la surface de la dent ;
b) à fabriquer un double de surmesure du modèle positif ;
c) à façonner le matériau céramique sur le double du modèle positif ;
d) à cuire le matériau céramique ;
e) à mettre en place l'élément de moulage céramique sur le modèle positif ;
f) à introduire un matériau de couche de protection plastiquement déformable et durcissant ou durcissable dans l'espace limité par l'élément de moulage céramique et le modèle positif ;
g) à faire durcir le matériau de la couche de protection.

28. Procédé selon la revendication 26, caractérisé en ce qu'on utilise pour la fabrication du double de surmesure, une empreinte négative du modèle positif avec une matière de moulage, qui lors de sa consolidation, subit une réduction de volume, et en ce que le double de surmesure est obtenu par coulage du moule négatif.

29. Procédé de fabrication d'un élément de restauration dentaire céramique selon l'une des revendications 1 à 18, caractérisé en ce qu'il comprend les étapes opératoires suivantes, consistant :
a) à fabriquer un modèle positif de la surface de la dent ;
b) à façonner le matériau céramique sur le modèle positif ;
c) à cuire le matériau céramique ;
d) à enlever le matériau céramique sur les segments de l'élément de moulage céramique qui correspondent aux surfaces subissant des contraintes de traction et/ou de cisaillement ou aux zones des bords des surfaces de l'élément de restauration dentaire céramique ;
e) à mettre en place l'élément de moulage céramique sur le modèle positif ;
f) à introduire un matériau de couche de protection plastiquement déformable et durcissant ou durcissable dans l'espace limité par l'élément de moulage céramique et le modèle positif ;
g) à faire durcir le matériau de la couche de protection.

30. Procédé de fabrication d'un élément de restauration dentaire céramique selon l'une des revendications 1 à 18, caractérisé en ce qu'il comprend les étapes opératoires suivantes, consistant :
a) à fabriquer un modèle positif de la surface préparée ;
b) à mesurer la surface préparée sur l'empreinte positive et à enregistrer le contour de surface mesuré ;
c) à former un élément de couche de protection ou un élément de maintien en place par commande numérique, dont une surface de séparation correspond au contour de surface enregistré et dont la seconde surface de séparation correspond à un second contour espacé, qui est déduit du premier contour de surface, par ex. par décalage parallèle ;
d) à façonner le matériau céramique sur la couche de protection formée ;
e) à cuire le matériau céramique.

31. Procédé de fabrication d'un élément de restauration dentaire céramique selon l'une des revendications 1 à 18, caractérisé en ce qu'il comprend les étapes opératoires suivantes, consistant :
a) à fabriquer un modèle positif de la surface préparée ;
b) à mesurer le modèle positif et à enregistrer son contour de surface ;
d) à façonner le matériau céramique sur un élément de matériau de couche de protection, lequel se compose de préférence de matériau mousse métallique ou céramique ;
et
d) à cuire le matériau céramique ;
e) à travailler une surface intérieure dans l'élément de matériau de couche de protection par commande numérique conformément au contour de surface enregistré ;
ou
d') à travailler une surface intérieure dans le matériau de couche de protection par commande numérique conformément au contour de surface enregistré ;
e') à cuire le matériau céramique.

32. Procédé selon la revendication 30 ou 31, caractérisé en ce que les pores de l'élément de matériau de couche de protection se composant de matériau mousse sont infiltrés au moins partiellement avec un matériau synthétique.

33. Procédé selon l'une des revendications 19 à 32, caractérisé en ce que les surfaces de l'élément de moulage céramique à recouvrir de matériau de couche de protection ont été enrichies en silicate par traitement de surface tribochimique ou décapées et/ou silanisées au moins partiellement.

34. Procédé selon l'une des revendications 19 à 33, caractérisé en ce que le modèle positif est doté d'un agent de séparation avant la mise en place de l'élément de moulage céramique et le façonnage du matériau de la couche de protection.

35. Procédé selon l'une des revendications 19 à 34, caractérisé en ce que le façonnage du matériau de la couche de protection est réalisé dans l'espace limité par l'élément de moulage céramique et l'empreinte positive sous l'action simultanée de la chaleur et/ou d'une oscillation mécanique, par ex. par ultrasons et/ou d'une compression.

36. Procédé selon l'une des revendications 19 à 35, caractérisé en ce que le matériau de la couche de protection se compose au moins pour partie de polymères de silicium organiques et est au moins partiellement céramisé.

37. Prothèse médicale, caractérisée en ce qu'elle comporte un volume de matériau mousse (52 ; 142), qui est imprégné au moins pour partie avec un matériau d'infiltration (60, 61 ; 58 ; 166).

38. Prothèse selon la revendication 37, caractérisée en ce que le volume de matériau mousse (52 ; 142) se compose d'un matériau mousse céramique ou métallique.

39. Prothèse selon la revendication 37 ou 38, caractérisée en ce que le matériau d'infiltration est un matériau synthétique (60 ; 166) ou un matériau céramique (61 ; 58).

40. Prothèse selon l'une des revendications 37 à 39, caractérisée en ce que le matériau d'infiltration est déformable élastiquement ou plastiquement.
